(19) Europäisches Patentamt
European Patent Office
Office européen des brevets

(11) Numéro de publication: **0 280 618 B1**

(12) **FASCICULE DE BREVET EUROPEEN**

(45) Date de publication de fascicule du brevet: **30.09.92**

(51) Int. Cl.5: **C07J 41/00**, C07J 1/00, C07J 21/00, C07J 31/00, C07J 51/00, A61K 31/56, A61K 31/58, C07J 43/00

(21) Numéro de dépôt: **88400371.6**

(22) Date de dépôt: **18.02.88**

# (54) Nouveaux 19-nor stéroîdes substitués en position 7, leur préparation, leur application comme médicaments, les compositions pharmaceutiques les renfermant.

(30) Priorité: **18.02.87 FR 8702072**

(43) Date de publication de la demande: **31.08.88 Bulletin 88/35**

(45) Mention de la délivrance du brevet: **30.09.92 Bulletin 92/40**

(84) Etats contractants désignés: **AT BE CH DE ES FR GB GR IT LI LU NL SE**

(56) Documents cités: **EP-A- 0 097 572**
**WO-A-83/03099**

(73) Titulaire: **ROUSSEL-UCLAF**
**35, Boulevard des Invalides**
**F-75007 Paris(FR)**

(72) Inventeur: **Nique, François**
**7, Allée Pierre Brossolette**
**F-93320 Pavillons-Sous-Bois(FR)**
Inventeur: **Nedelec Lucien**
**45, Boulevard de l'Ouest**
**F-93340 Le Raincy(FR)**
Inventeur: **Bouton, Marie-Madeleine**
**47-49, Avenue du Docteur Arnold Netter**
**F-75012 Paris(FR)**
Inventeur: **Philibert, Daniel**
**16, rue Chevalier**
**F-94210 La Varenne Saint-Hilaire(FR)**

(74) Mandataire: **Bourgouin, André et al**
**Département des Brevets ROUSSEL UCLAF**
**111, route de Noisy B.P. no 9**
**F-93230 Romainville(FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).



Rank Xerox (UK) Business Services

## Description

La présente invention concerne de nouveaux 19-nor stéroïdes substitués en position 7, leur préparation, leur application comme médicaments, les compositions pharmaceutiques les renfermant.

L'invention a pour objet les produits de formule générale (I) :

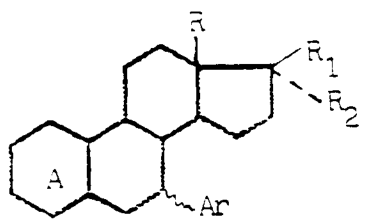

(I)

dans laquelle le cycle A présente l'une des structures suivantes :

a/. soit A représente le groupement :

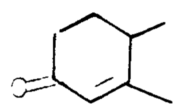

b/. soit A représente le groupement :

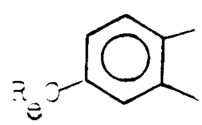

dans lequel Re représente un atome d'hydrogène, un radical alkyle ayant de 1 à 6 atomes de carbone éventuellement substitué ou un radical acyle,

R représente un radical méthyle ou éthyle,

$R_1$ représente un radical hydroxyle éventuellement protégé ou acylé par un radical acyle choisi parmi les radicaux acétyle, chloroacétyle ou trifluoroacétyle, ou $R_1$ représente un radical alcoxyle, $R_2$ représente un atome d'hydrogène, un radical alkyle, alkényle ou alkynyle ayant au plus 8 atomes de carbone ou un radical aryle ou aralkyle ayant au plus 15 atomes de carbone, chacun de ces radicaux alkyle, alkényle, alkynyle, aryle ou aralkyle étant éventuellement substitué, par un ou plusieurs substituants identiques ou différents choisis parmi les radicaux :

- halogène,
- alkoxy,
- alkylthio,
- amino, alkylamino, dialkylamino, chacun des radicaux dialkylamino étant éventuellement sous forme oxydée,
- aminoalkyl,
- dialkylaminoalkyle,
- dialkylaminoalkyloxy,
- hydroxyle, acétoxy, un radical de formule

$$-O-\underset{\underset{O}{\|}}{C}-(CH_2)_nCO_2H$$

dans lequel n = 2 à 5,

- acyle,
- carboxy libre ou estérifié,
- cyano,
- trifluorométhyle,
- aryle ou aralkyle,

les radicaux aryle et aralkyle, que peut représenter $R_2$ pouvant être de plus substitués par un radical alkyle,

soit $R_1$ et $R_2$ forment ensemble un radical choisi parmi les radicaux :

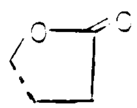 , 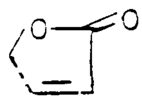 ; 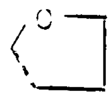 ; 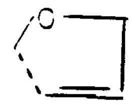

Ar représente un radical aryle ayant 5 ou 6 chaînons carbocyclique ou hétérocyclique, choisi parmi les radicaux thiényle, furyle, thiazolyle, pyrrolyle, oxazolyle, imidazolyle, pyrazolyle, triazolyle (1,2,3 ou 1,2,4) tétrazolyle, isothiazolyle, isoxazolyle, thiadiazolyle, phényle, pyridinyle, pyridazinyle, pyrimidinyle, pyrazinyle, pipéradinyle,

ces radicaux étant éventuellement substitués par un ou plusieurs substituants identiques ou différents choisis parmi les radicaux :

- halogène,
- alkyle,
- alkoxy,
- alkylthio,
- amino, alkylamino, dialkylamino, chacun des radicaux dialkylamino étant éventuellement sous forme oxydée,
- aminoalkyl,
- dialkylaminoalkyle,
- dialkylaminoalkyloxy,
- hydroxyle, acétoxy, un radical de formule

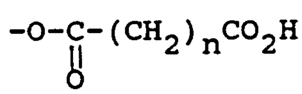

  dans lequel n = 2 à 5,
- acyle,
- carboxy libre ou estérifié,
- cyano,
- trifluorométhyle,
- aryle ou aralkyle,

ainsi que les sels d'addition des produits de formule (I) avec les acides et les bases.

Par alkyle, on entend particulièrement les radicaux méthyle, éthyle, propyle, isopropyle, butyle, sec-butyle, tert-butyle, pentyle, hexyle, 2-méthylpentyle, 2,2-diméthylpentyle.

Par alkényle, on entend plus particulièrement les radicaux vinyle, propényle, allyle, isopropényle, 2-méthylallyle, isobutényle ou butényle.

Par alkynyle, on entend plus particulièrement les radicaux éthynyle, propynyle, propargyle ou isobutynyle ou butynyle.

Par acyle, on entend en particulier acétyle, propionyle, butyryle, benzoyle.

Par alcoxyle, on entend plus particulièrement les radicaux dérivés des radicaux alkyle mentionnés ci-dessus et en particulier les radicaux méthoxy, éthoxy, propyloxy, isopropyloxy, butyloxy.

Dans la formule générale et dans ce qui suit, l'expression aryle comprend en général les radicaux carbocycliques ou hétérocycliques.

Parmi les radicaux à 5 chaînons, on peut citer les radicaux thiényle, furyle, thiazolyle, pyrrolyle, oxazolyle, imidazolyle, pyrazolyle, triazolyle (1,2,3 ou 1,2,4) tétrazolyle, isothiazolyle, isoxazolyle.

Parmi les radicaux à 6 chaînons, on peut citer les radicaux phényle, pyridinyle, pyridazinyle, pyrimidinyle, pyrazinyle.

Parmi les radicaux préférés, par aryle on entend plus particulièrement les radicaux phényle, furyle, thiényle et par aralkyle, on entend plus particulièrement le radical benzyle, ces radicaux étant eux-mêmes éventuellement substitués par des radicaux alkyle, alkoxy, alkylthio, aminoalkyl ou dialkylamino indiqués ci-dessus.

L'expression "éventuellement substitué" appliquée aux radicaux alkyle, alkényle, alkynyle, aryle ou aralkyle comprend de préférence les radicaux suivants :

- halogène : fluor, chlore, brome, iode,
- alkyle tel que méthyle, éthyle, propyle, isopropyle, butyle, isobutyle, tert-butyle pour les radicaux aryle et aralkyle,
- alkoxy tel que méthoxy, éthoxy, propyloxy, isopropyloxy, butyloxy,
- alkylthio tel que méthylthio, éthylthio, propylthio, isopropylthio, butylthio,
- amino, alkylamino tel que méthylamino ou éthylamino, dialkylamino tel que diméthylamino, diéthylamino, méthyléthylamino, chacun des radicaux dialkylamino étant éventuellement sous forme oxydée,
- amino alkyl tel que aminométhyle ou aminoéthyle,
- dialkylaminoalkyle tel que diméthylaminométhyle ou diméthylaminoéthyle,
- dialkylaminoalkyloxy tel que diméthylaminoéthyloxy,
- hydroxyle éventuellement acylé, par exemple acétoxy ou un radical de formule

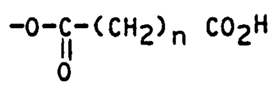

  dans lequel n = 2 à 5 notamment,
- acyle tel que acétyle, propionyle, butyryle, benzoyle,
- carboxy libre, estérifié tel que alkoxy carbonyle par exemple méthoxy carbonyle ou éthoxy carbonyle,
- cyano,
- trifluorométhyle,
- aryle ou aralkyle éventuellement substitués.

Bien entendu, l'expression "éventuellement substituée" indique qu'un ou plusieurs substituants identiques ou différents peuvent être présents.

Lorsque $R_e$ comporte un substituant, il s'agit de préférence du substituant amino ou dialkylamino tel que diméthylamino.

L'expression "hydroxyle éventuellement protégé" désigne les groupements protecteurs classiques tels que les groupements acyle comme acétyle, chloroacétyle, trifluoroacétyle, les groupements tétrahydropyrannyle, les groupements silyle tels que triméthylsilyle, tert-butyl diméthylsilyle.

Selon la valeur des substituants portés par les radicaux $R_2$ et Ar, les produits de formule (I) peuvent former des sels avec les acides ou les bases ; c'est ainsi que si l'un au moins des radicaux $R_2$ ou Ar comporte une fonction amino salifiable, les produits de formule (I) peuvent former des sels avec les acides. Il peut alors s'agir des sels, des acides chlorhydrique, bromhydrique, nitrique, sulfurique, phosphorique, acétique, formique, propionique, benzoïque, maléique, fumarique, succinique, tartrique, citrique, oxalique, glyoxylique, aspartique, alcane sulfoniques tels que les acides méthane et éthanesulfoniques, arylsulfoniques, tels que les acides benzène et para-toluènesulfoniques et arylcarboxyliques.

Par contre, si l'un au moins des radicaux $R_2$ ou Ar comporte une fonction carboxy, elle peut être salifiée par un reste de base. On peut alors avoir un sel de sodium, de potassium, de lithium, de calcium, de magnésium ou d'ammonium. On peut citer, parmi les bases organiques, la méthylamine, la propylamine, la triméthylamine, la diéthylamine, la triéthylamine, la N,N-diméthyléthanolamine, le tris (hydroxyméthyl) amino méthane, l'éthanolamine, la pyridine, la picoline, la dicyclohexylamine, la morpholine, la benzylamine, la procaïne, la lysine, l'arginine, l'histidine, la N-méthylglucamine.

La présente invention a notamment pour objet les produits de formule (I) telle que définie ci-dessus dans laquelle le cycle A représente le groupement :

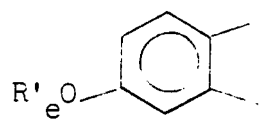

dans lequel $R'_e$ représente un atome d'hydrogène ou un radical alkyle ayant de 1 à 4 atomes de carbone.

La présente invention a particulièrement pour objet les produits de formule (I) telle que définie ci-dessus dans laquelle Ar représente :

a/. soit un radical phényle éventuellement substitué par un ou plusieurs substituants choisis parmi les radicaux halogène, alkyle, alkoxy ou alkylthio ayant de 1 à 4 atomes de carbone, amino, alkylamino, dialkylamino, dialkylamino alkoxyle, hydroxyle, acyle, carboxy libre estérifié ou salifié, cyano, trifluorométhyle, phényle ou benzyle eux-mêmes éventuellement substitués par un ou plusieurs radicaux alkyles ayant de 1 à 4 atomes de carbone,

b/. soit un radical hétérocyclique choisi parmi les radicaux thiényle, furyle, thiazolyle, isothiazolyle, oxazolyle, isoxazolyle, thiadiazolyle, pyridinyle, pipéridinyle.

L'invention a plus particulièrement pour objet les produits de formule (I) telle que définie ci-dessus dans laquelle $R_1$ représente un radical hydroxyle éventuellement protégé ou acylé, $R_2$ représente un radical alkyle, alkényle ou alkynyle, chacun de ces radicaux étant éventuellement substitué par un radical choisi parmi les halogènes, les radicaux hydroxyle, carboxy éventuellement estérifié ou salifié ou cyano.

L'invention a spécialement pour objet, les produits décrits ci-après dans la partie expérimentale et en particulier :

- Le 7alpha-[4-[2-(diméthylamino) éthoxy] phényl] estra-1,3,5(10)-trièn-3,17béta-diol
- Le 7alpha-[4-[2-(diméthylamino) éthoxy] phényl] 17béta-hydroxy estr-4-èn-3-one.
- Le 7béta-[4-[2-(diméthylamino) éthoxy] phényl] estra-1,3,5(10)-trièn-3,17béta-diol.
- La 7béta-[4-[2-(diméthylamino) éthoxy] phényl] 17béta-hydroxy estr-4-èn-3-one.
- Le 7alpha -[4-(diméthylamino) phényl] estra-1,3,5(10)-trién-3,17béta-diol.
- Le 7alpha -(4-méthoxy phényl) estra-1,3,5(10)-trièn-3,17béta-diol.
- La 17béta-hydroxy 7alpha-[4-(méthylthio) phényl] estr-4-èn-3-one.
- Le 7alpha -[4-(méthylthio) phényl] estra-1,3,5(10)-trièn-3,17béta-diol.

L'invention a également pour objet un procédé de préparation des produits de formule (I) telle que définie ci-dessus caractérisé en ce l'on soumet un produit de formule (II) :

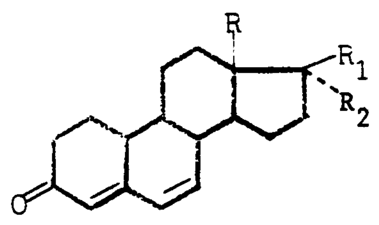

(II)

dans laquelle R, $R_1$ et $R_2$ ont la signification indiquée ci-dessus d'abord à l'action, éventuellement en présence d'un acide de Lewis,

- soit d'un produit de formule ($III_a$) :

  Ar-Mg-Hal ($III_a$)

  dans laquelle Ar a la signification indiquée ci-dessus et Hal représente un atome d'halogène en présence d'un sel cuivreux :

- soit d'un produit de formule ($III_b$) :

  $Ar_2CuLi$ ($III_b$)

- soit d'un produit de formule ($III_c$) :

  $Ar_2CuCNLi_2$ ($III_c$)

pour obtenir un produit de formule ($I_A$) :

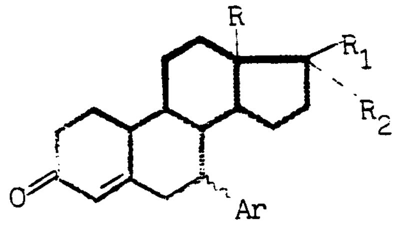

$(I_A)$

éventuellement sous forme d'un mélange d'isomères 7alpha et 7béta que l'on sépare, si désiré et produit de formule ($I_A$) correspondant à un produit de formule (I) dans laquelle le cycle A représente le groupement :

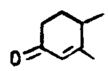

que l'on traite, si désiré par un agent d'aromatisation pour obtenir un produit de formule

$(I_{B_1})$ :

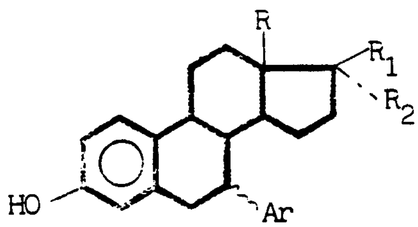

$(I_{B_1})$

dans laquelle Ar, R, $R_1$ et $R_2$ ont la signification indiquée ci-dessus et correspondant à un produit de formule (I) dans laquelle le cycle A représente le groupement :

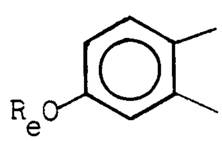

Re représentant un atome d'hydrogène et produit de formule

$(I_{B_1})$

que l'on traite éventuellement par un réactif d'alkylation pour obtenir un produit de formule

$(I_{B_2})$ :

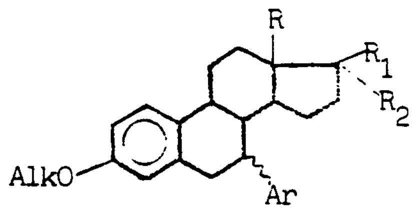

$(I_{B_2})$

dans laquelle Alk représente un radical alkyle ayant de 1 à 6 atome de carbone éventuellement substitué et correspondant à un produit de formule (I) dans lequel le cycle A représente le groupement :

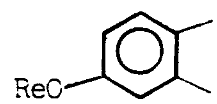

Re représentant un radical alkyle éventuellement substitué, produits de formules $(I_A)$,

$(I_{B_1})$ ou $(I_{B_2})$

que, selon la valeur des substituants $R_2$ et Ar l'on soumet éventuellement à l'action d'une base ou d'un acide pour obtenir les sels correspondants.

Dans un mode préféré d'exécution du procédé, Hal représente un atome de chlore, de brome ou d'iode, de préférence de brome. Le sel cuivreux que l'on utilise est le chlorure, le bromure, l'iodure ou le cyanure ou le complexe bromure cuivreux-diméthyl sulfure cuivreux. Le milieu réactionnel peut ensuite être soumis à un acide fort tel que l'acide chlorhydrique, l'acide nitrique ou l'acide sulfurique.

Le sel cuivreux est lui-même de préférence préparé in situ par réaction notamment du chlorure cuivrique avec le bromure de lithium. L'acide de Lewis éventuellement utilisé peut être le trifluorure de bore.

Lorsque l'on utilise un cupro-lithien de formule $Ar_2CuLi$, celui-ci est également de préférence préparé in situ par action de l'iodure cuivreux sur le produit de formule Ar-Li, lui-même préparé à partir de l'halogénure Ar-Hal.

Le produit de formule $(III_c)$ peut être préparé par action du cyanure cuivreux sur le produit de formule Ar-Li.

L'agent d'aromatisation que l'on utilise de préférence pour transformer les produits de formule $(I_A)$ en produit de formule

$(I_{B_1})$

peut être choisi parmi les réactifs suivants :

a/. On traite d'abord le produit de formule $(I_A)$ par un agent d'halogénation, puis de deshydrohalogénation, tel que le réactif bromure cuivreux, bromure de lithium dans un solvant tel que l'acétonitrile.

b/. On traite le produit de formule $(I_A)$ par une base forte tel que le butyl lithium, on bloque l'énolate lithien formé par un réactif tel que le chlorure de tert-butyl diméthyl silyle puis déshydrogène le produit ainsi obtenu par un réactif tel que la 2,3-dichloro 5,6-dicyano benzoquinone.

L'alkylation des produits de formule

$(I_{B_1})$

pour obtenir les produits de formule

$(I_{B_2})$

est réalisée de façon usuelle. On opère par exemple à l'aide d'un halogénure ou d'un sulfate d'alkyle. On

utilise de préférence l'iodure d'alkyle.

Lorsque l'on veut séparer les deux isomères de formule ($I_A$), on opère dans les conditions usuelles en utilisant les techniques de cristallisation ou de chromatographie.

L'éventuelle formation des sels avec les acides ou les bases est également effectuée dans les conditions usuelles.

L'invention a également pour objet un procédé de préparation des produits de formule (I), caractérisé en ce que l'on soumet un produit de formule ($I_C$) :

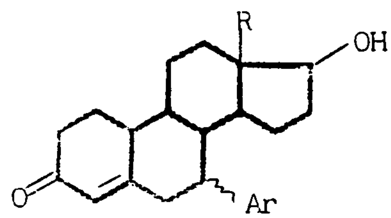

($I_C$)

dans laquelle R et Ar ont la signification précédente, correspondant à un produit de formule ($I_A$) dans laquelle $R_1$ représente un radical hydroxyle et $R_2$ représente un atome d'hydrogène, à l'action d'un réactif de blocage de la fonction 3-céto par exemple-un éther d'énol ou un cétal-pour obtenir un produit de formule (IV) :

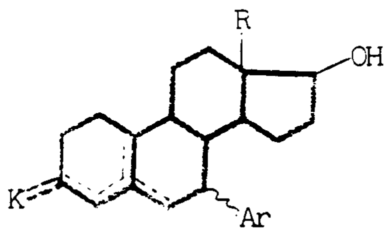

(IV)

dans laquelle K et les traits pointillés représentent une fonction cétonique protégée, de préférence un produit de formule ($IV_A$) ou ($IV_B$) :

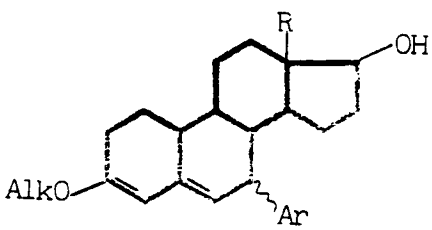

($IV_A$)

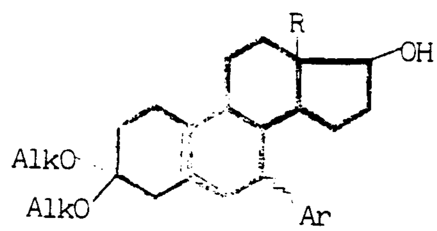

($IV_B$)

dans laquelle Alk représente de préférence un radical alkyle et les traits pointillés représentent une double liaison en 5(6) ou 5(10), produit de formule (IV) que l'on traite par un agent d'oxydation pour obtenir un produit de formule (V) :

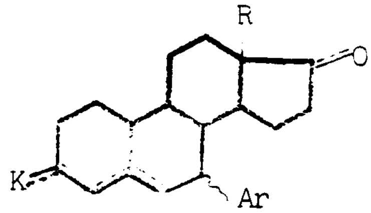

(V)

produit sur lequel on fait agir soit un dérivé organo métallique dérivé d'un radical $R_2$ représentant un radical alkyle, alkényle, alkynyle ayant au plus 8 atomes de carbone ou un radical aryle ou aralkyle ayant au plus 15 atomes de carbone, chacun de ces radicaux alkyle, alkényle, alkynyle, aryle ou aralkyle étant éventuellement substitué, soit d'abord un dérivé organométallique dérivé du réactif de formule $-C\equiv C-CH_2OH$ dans lequel la fonction hydroxyle est éventuellement protégée, puis dans un ordre quelconque éventuellement à un réactif de déprotection et à un réactif de réduction totale ou partielle, puis à un réactif de cyclisation ou d'oxydation, soit d'abord à un réactif de formule

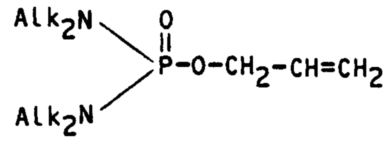

en présence d'une base forte pour obtenir respectivement les produits de formule ($VI_A$) :

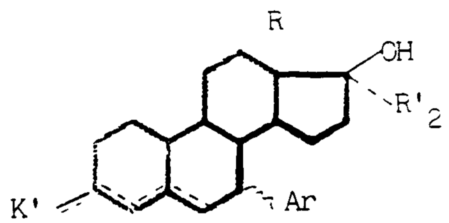

($VI_A$)

dans laquelle $R'_2$ a les valeurs de $R_2$ à l'exception de la valeur hydrogène et K' et les traits pointillés représentent une fonction 3-céto delta 4 éventuellement protégée et les produits de formule ($VI_B$) :

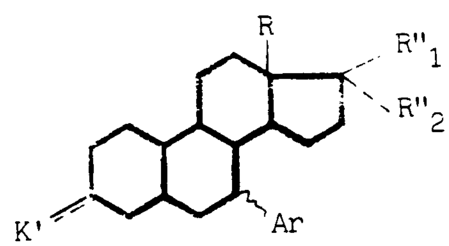

($VI_B$)

dans laquelle $R''_1$ et $R''_2$ représentent ensemble un radical :

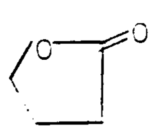 , 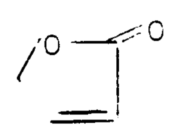 ; 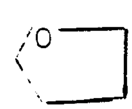 ou 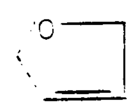

produits de formules ($VI_A$) et ($VI_B$) que l'on soumet éventuellement à un réactif de déprotection lorsque K' représente un groupement protecteur de la fonction 3-céto delta-4 pour obtenir les produits de formule ($I_D$) :

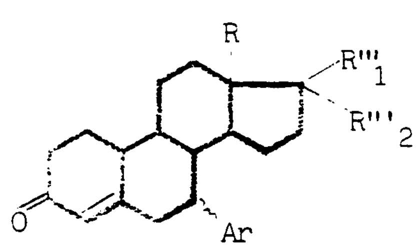

($I_D$)

dans laquelle $R'''_1$ et $R'''_2$ ont les valeurs indiquées ci-dessus pour $R_1$ et $R_2$ à l'exception de la valeur $R_2$ = hydrogène.

Les produits de formules ($I_D$) dans laquelle $R''_1$ = OH peuvent éventuellement être soumis à un réactif de protection d'acylation ou d'alkylation de la fonction 17béta-OH. Les produits de formule ($I_D$) ainsi que les produits ($I'_D$) correspondants dans lesquels la fonction 17 béta-OH a été protégée acylée ou alkylée constituent les produits de formule ($I_A$) qui peuvent ensuite être traités comme indiqué ci-dessus.

La protection de la fonction 3-céto delta-4 du produit de formule ($I_C$) est effectuée de préférence sous forme d'un éther d'énol par action notamment d'un orthoformiate d'alkyle tel que l'orthoformiate d'éthyle en présence d'acide para-toluènesulfonique.

L'agent d'oxydation que l'on fait agir sur le produit de formule (IV) pour obtenir un produit de formule (V) peut être choisi parmi le réactif de Jones, le dichromate de pyridinium ou le chlorochromate de pyridinium. On peut également utiliser la réaction d'oxydation d'Oppenauer en présence de cyclohexanone et d'isopropylate d'aluminium dans le toluéne au reflux.

L'organométallique que l'on fait agir sur le produit de formule (V) est de préférence un magnésien. Dans le cas où le radical alkyle, alkényle, alkynyle, aryle ou aralkyle à introduire comporte une fonction réactive, cette fonction peut être protégée par les méthodes usuelles. La réaction est conduite selon les méthodes usuelles. On opère de préférence en présence d'un halogénure de cérium tel que $CeCl_3$ lorsque le radical $R_2$ représente un radical aryle.

Il en est de même de l'addition d'un réactif de formule $-C{\equiv}C-CH_2OH$ dont la fonction hydroxyle est de préférence protégée par un réactif tel que le tétrahydropyrannyle ou le tert-butyle.

Le réactif de réduction totale qui conduit au substituant de formule $-(CH_2)_3-OH$ est l'hydrogène en présence d'un catalyseur tel que le palladium sur charbon ou un catalyseur au rhodium tel que le réactif de Wilkinson.

Pour obtenir une réduction partielle conduisant au substituant de formule $-CH=CH-CH_2OH$, on utilise l'hydrogène avec un catalyseur empoisonné tel que le palladium sur sulfate de baryum empoisonné par la pyridine ou la triéthylamine.

La réaction de cyclisation qui conduit aux produits comportant un substituant :

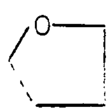
ou
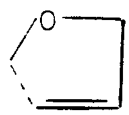

en position 17
est réalisée en présence de chlorure de paratoluène sulfonyle et de pyridine.

La réaction d'oxydation qui conduit in situ à la cyclisation et à l'obtention des produits comportant un substituant :

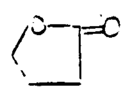
ou
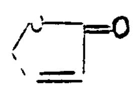

en position 17
est réalisée à l'aide des agents d'oxydation mentionnés précédemment.

L'action du tétraalkyl phosphorodiamidate d'allyle, de préférence le tétraméthyle est effectuée selon la méthode décrite par STURTZ et YAOUANC dans Synthésis 1980 p. 289. La base forte, en présence de laquelle on agit, est de préférence le butyllithium. On peut également opérer en présence de diazabicyclooctane (DABCO) ou d'un éther couronné.

Les produits de formules ($VI_A$) et ($VI_B$) peuvent, selon la valeur de K', être des produits de formule (I). Lorsque K' représente K, c'est-à-dire un groupement 3-céto delta-4 protégé, la déprotection peut être réalisée par les méthodes usuelles, notamment en milieu acide tel que l'acide chlorhydrique aqueux.

Les réactions de protection, d'acylation ou d'alkylation de la fonction 17béta-OH sont réalisées selon les méthodes usuelles. On opère, par exemple, à l'aide d'un halogénure d'acyle tel que le chlorure d'acétyle ou un anhydride mixte ou symétrique tel que l'anhydride acétique.

Les produits de formule (I) ainsi que leurs sels d'addition avec les acides et les bases pharmaceutiquement acceptables sont des produits particulièrement intéressants du point de vue pharmacologique ; ils possèdent, en particulier, des propriétés anti-prolifératives, anti-estrogènes et/ou estrogènes.

Ces propriétés les rendent utilisables dans le traitement des carcinomes hormono-dépendants, comme par exemple les carcinomes mammaires et ses métastases. Ces propriétés rendent les produits de formule (I) ainsi que leurs sels d'addition avec les acides et les bases pharmaceutiquement acceptables également utilisables dans le traitement des tumeurs bénignes du sein.

Les propriétés estrogènes que peuvent également présenter lesdits produits de formule (I) ainsi que leurs sels d'addition avec les acides et les bases pharmaceutiquement acceptables les rendent utilisables également dans le traitement des troubles liés à une hypofolliculinie, par exemple les aménorrhées, les dysménorrhées, les avortements répétés, les troubles prémenstruels ainsi que le traitement de la ménopause.

L'invention a donc pour objet, à titre de médicaments, les produits de formule (I) ainsi que leurs sels d'addition avec les acides et les bases pharmaceutiquement acceptables, c'est-à-dire non toxiques aux doses utilisées.

L'invention a plus spécialement pour objet, à titre de médicaments, les produits de formule (I) telle que définie ci-dessus dans laquelle $R_1$ représente un radical hydroxyle éventuellement protégé ou acylé, $R_2$ représente un radical alkyle, alkényle ou alkynyle, chacun de ces radicaux étant éventuellement substitué par un radical choisi parmi les halogènes, les radicaux hydroxyle, carboxy éventuellement estérifié ou salifié ou cyano.

L'invention a plus particulièrement pour objet, à titre de médicament, les produits de formule (I) décrits ci-après dans les exemples et spécialement :

- Le 7alpha-[4-[2-(diméthylamino) éthoxy] phényl] estra-1,3,5(10)-trièn-3,17béta-diol.
- Le 7alpha-[4-[2-(diméthylamino) éthoxy] phényl] 17béta-hydroxy estr-4-èn-3-one.
- Le 7béta-[4-[2-(diméthylamino) éthoxy] phényl] estra-1,3,5(10)-trièn-3,17béta-diol.
- La 7béta-[4-[2-(diméthylamino) éthoxy] phényl] 17béta-hydroxy estr-4-èn 3-one.
- Le 7alpha-[4-(diméthylamino) phényl] estra-1,3,5(10)-trièn 3,17béta-diol.
- Le 7alpha -(4-méthoxyphényl) estra-1,3,5(10)-triène-3,17 béta-diol.
- La 17béta-hydroxy 7alpha-[4-(méthylthio) phényl] estr-4-èn-3-one.
- Le 7alpha-[4-[méthylthio) phényl] estra-1,3,5(10)-trièn-3,17béta-diol.

La posologie utile varie en fonction de l'affection à traiter et de la voie d'administration ; elle peut varier par exemple de 1 mg à 1 g et de préférence de 1 à 100 mg par jour chez l'adulte par voie orale.

Les nouveaux produits de formule (I), et leurs sels, tels que définis ci-dessus peuvent être employés pour préparer des compositions pharmaceutiques renfermant, à titre de principe actif, l'un au moins desdits produits.

Les produits de formule (I) et leurs sels sont utilisés par voie digestive, parentérale ou locale. Ils peuvent être prescrits sous forme de comprimés simples ou dragéifiés, de gélules, de granulés, de suppositoires, d'ovules, de préparations injectables, de pommades, de crèmes, de gels, lesquels sont préparés selon les méthodes usuelles.

Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émusifiants, les conservateurs.

L'invention a donc pour objet les compositions pharmaceutiques renfermant comme principe actif au moins un produit de formule (I).

Les produits de formule (II) utilisés au départ du procédé de préparation des produits de formule (I) sont connus ou peuvent être préparés, par des méthodes telles que celles décrites ci-dessus pour le passage des produits de formule ($I_C$) aux produits de formule ($I_D$), à partir du produit de formule :

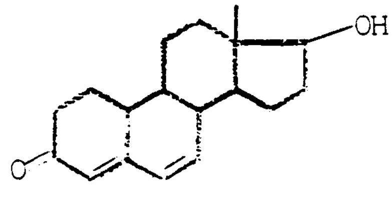

décrit dans les brevets américains USP 2,739,974 et 3.099.664.

Les exemples suivants illustrent l'invention sans toutefois la limiter.

**Exemple 1 : 17 beta-acétoxy 7 béta-[4[2-(diméthylamino) éthoxy) phényl] estr-4-èn-3-one et 17 béta-acétoxy 7-alpha [4-[2-(diméthylamino) ethoxy] phényl] estr-4-èn-3-one.**

1¤/. Préparation du magnésien :

On mélange, sous atmosphère inerte, 1,32 g de magnésium en copeaux, 6 cm3 de tétrahydrofuranne anhydre, introduit à 40¤C, en 50 minutes environ, après amorçage par 2 gouttes de dibromoéthane, 11 g de 4-[2-(diméthylamino) éthoxy] bromobenzène en solution dans 30 cm3 de tétrahydrofuranne anhydre, agite pendant 2 heures à 40¤C, note une cristallisation.

2¤/. Addition 1-6 :

On ajoute dans la suspension de magnésien obtenu au paragraphe 1 1,5 g de chlorure cuivreux anhydre, agite à 20¤C pendant 30 minutes, introduit à -35¤C en 45 minutes environ 6,6 g de 17 béta-acétoxy estra 4,6-dièn 3-one en solution dans 30 cm3 de tétrahydrofuranne anhydre, acidifie au bout d'une heure la suspension réactionnelle par 20 cm3 d'une solution aqueuse 5 N d'acide chlorhydrique, verse sur une solution aqueuse saturée de bicarbonate de sodium, extrait à l'acétate d'éthyle, lave par une solution aqueuse de chlorure d'ammonium, à l'eau salée, sèche, concentre à sec par distillation sous pression réduite, chromatographie le résidu (12,6 g) sur silice en éluant sous pression d'azote par un mélange de chlorure de méthylène, d'isopropanol et d'ammoniaque (95/5/0,5) puis par un mélange de chlorure de méthylène, disopropanol et d'ammoniaque (90/10/1) et obtient 5,62 g de 17 béta-acétoxy 7 béta-[4-[2-(diméthylamino) éthoxy] phényl] estr-4-èn-3-one (produit I) 2,53 g de 17 béta-acétoxy 7 alpha-[4-[2-(diméthylamino) éthoxy] phényl estr-4-èn-3-one (produit II).

**Spectre de RMN ($CDCl_3$) en ppm :**

| | |
|---|---|
| H du Me en 18 | 0,82 (s) |
| H de O AC | 2,02 (s) |
| H de 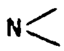 | 2,33 (s) |
| H de 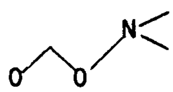 | 2,73 (t) |
| H de 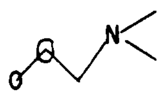 | 4,06 (t) |
| 17 alpha H | 4,49 (t) |
| H4 | 5,81 |

H de 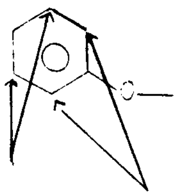

7,08(d) 6,84(d)

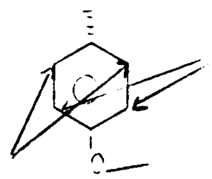

6,74-6,84

7,03-7,13

<u>Spectre de RMN ($CDCl_3$) en ppm</u> :

| | |
|---|---|
| H du Me en 18 | 0,88 (s) |
| H de OAC | 2,02 (s) |
| H de 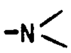 | 2,35 (s) |
| H de 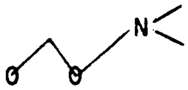 | 2,73 (t) |
| 7 bêta H | 3,06 |
| H de 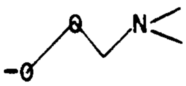 | 4,07 (t) |
| 17 alpha H | 4,43 (t) |
| $H_4$ | 5,88 |

**<u>Exemple 2 : 17 béta-acétoxy 7 alpha-[4-[2-(diméthylamino) éthoxy] phényl] estra-1,3,5(10)-trièn-3-ol</u>**

On mélange, sous atmosphère inerte, 2,33 g de produit II (7 alpha) obtenu à l'exemple 1, 100 cm3 d'acétonitrile, 3,44 g de bromure cuivrique, 470 mg de bromure de lithium, agite à 75¤C pendant 1 heure et 45 minutes, refroidit à 20¤C, alcalinise par du bicarbonate de sodium, extrait au chloroforme, lave par une solution aqueuse de chlorure d'ammonium, sèche, concentre à sec par distillation sous pression réduite, chromatographie le résidu (2,4 g) sur silice en éluant par un mélange d'acétate d'éthyle et de triéthylamine (8/2) et obtient 1,18 g de produit attendu.

<u>Spectre de RMN ($CDCl_3$) en ppm</u> :

| | | |
|---|---|---|
| H de Me en 18 | 0,84 (s) | |
| H de OAC | 2,03 (s) | |
| H de 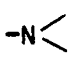 | 2,35 (s) | |
| H de 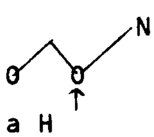 | 2,75 | |
| 7 béta H | 3,07 | $J_{H7-H8}$ ~ 3 Hz |
| H de 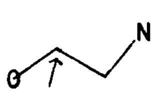 | 4,04 | |
| H aromatiques | 6,63 à 7,20 | |

**<u>Exemple 3 : 7 alpha -[4-[2-(diméthylamino) éthoxy] phényl] estra-1,3,5 (10)-trièn-3,17 béta-diol</u>**

On mélange, sous atmosphère inerte, 1,18 g de produit obtenu à l'exemple 2, 12 cm3 de méthanol, 3,7 cm3 de solution méthanolique, 2M de potasse, agite la solution obtenue à 20¤C pendant 1 heure, concentre à sec par distillation sous pression réduite, acidifie par 4 cm3 d'une solution aqueuse 2N d'acide chlorhydrique, alcalinise par du bicarbonate de sodium, extrait à l'acétate d'éthyle, lave à l'eau salée, sèche, concentre à sec par distillation sous pression réduite, chromatographie le résidu (1,08 g) sur silice en éluant par un mélange d'acétate d'éthyle et de triéthylamine (8/2) et obtient 1,02 g de produit attendu :
- $[alpha]_D$ = +1,5 ¤(1% chloroforme)

<u>Spectre de RMN ($CDCl_3$) en ppm</u> :

| | |
|---|---|
| H de Me en 18 | 0,78 (s) |
| H de N< | 2,35 (s) |
| H de 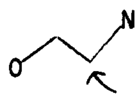 | 2,75 (t) |
| 17 alpha H | 3,60 |
| H de 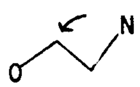 | 4,02 (t). |
| H aromatique | de 6,61 à 7,23 |

## Exemple 4 : 7 alpha - [4-(2- diméthylamino) éthoxy] phényl] 17 béta-hydroxy estr-4-èn-3-one

On mélange, sous atmosphère inerte, 480 mg de produit II (7 alpha) obtenu à l'exemple 1 (soit du 17 béta-acétoxy 7 alpha-[4-[2-(diméthylamino) éthoxy] phényl] estr-4-èn-3-one), 5 cm3 de méthanol, 1 cm3 de solution méthanolique, 2M de potasse, agite la solution obtenue à 20¤C pendant 3 heures et 30 minutes, élimine le méthanol par distillation sous pression réduite, ajoute de l'eau au résidu, extrait à l'acétate d'éthyle, au chlorure de méthylène, lave à l'eau salée les phases organiques, sèche, concentre à sec par distillation sous pression réduite, chromatographie le résidu (340 mg) sur silice en éluant avec un mélange d'acétate d'éthyle et de triéthylamine (9/1) et obtient 160 mg de produit attendu.

On chromatographie 320 mg (provenant d'une opération) de produit ainsi obtenu sur 90 g de lichroprep. Si 60 (5-20 Um) en éluant par un mélange d'acétate d'éthyle et de triétylamine (9/1) et obtient 180 mg de produit attendu pur.

<u>Spectre de RMN ($CDCl_3$) en ppm</u> :

| | |
|---|---|
| H de Me en 18 | 0,83 (s) |
| H de -N< | 2,32 (s) |

| | |
|---|---|
| H de 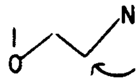 | 2,71 (t) |
| H de 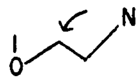 | 4,04 (t) |
| $H_4$ | 5,88 |
| 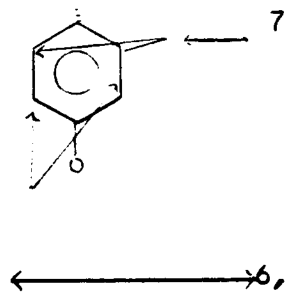 | 7,06 (d) |
| | 6,82 (d) |

**Exemple 5 : 17 béta-acétoxy 7 béta-[4-[2-(diméthylamino)} éthoxy] phényl] estra-1,3,5, (10)-trièn 3-ol.**

On mélange, sous atmosphère inerte, 2,4 g de produit I (7 béta) obtenu à l'exemple 1, 100 cm3 d'acétonitrile, 3,7 g de bromure cuivrique, 500 mg de bromure de lithium, agite à 75¤C pendant 1 heure et 30 minutes, ramène la température à 20¤C, alcalinise par du bicarbonate de sodium, extrait au chloroforme, lave par une solution aqueuse de chlorure d'ammonium, sèche, concentre à sec par distillation sous pression réduite, chromatographie le résidu (2,55 g) sur silice en éluant par un mélange d'acétate d'éthyle et de triéthylamine (8/2) et obtient 800 mg de produit attendu.

Spectre de RMN ($CDCl_3$) en ppm :

| | |
|---|---|
| H du Me en 18 | 0,76 (s) |
| H de OAc | 2,02 (s) |
| H de N< | 2,32 (s) |
| H de 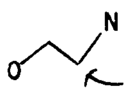 | 2,71 (t) |
| H de 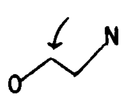 | 4,03 (t) |
| H aromatiques | de 6,58 à 7,21 |

**Exemple 6 : 7 béta-[4-[2-(diméthylamino) éthoxy] phényl] estra-1,3,5(10)-trièn 3,17 béta-diol**

On mélange, sous atmosphère inerte, 800 mg de produit obtenu à l'exemple 5, 8 cm3 de méthanol, 2,5 cm3 de solution méthanolique 2M de potasse, agite la solution obtenue à 20¤C pendant 3 heures et 30 minutes, élimine le méthanol par distillation sous pression réduite, acidifie par une solution aqueuse 2N d'acide chlorhydrique, alcalinise par du bicarbonate de sodium, extrait à l'acétate d'éthyle, lave à l'eau salée, sèche, concentre à sec par distillation sous pression réduite, chromatographie le résidu (720 mg) sur silice en éluant par un mélange d'acétate d'éthyle et de triéthylamine (8/2) et obtient 660 mg de produit attendu.

$[alpha]_D$ = +25 ¤ (c=1%, chloroforme).

Spectre de RMN ($CDCl_3$) en ppm :

| | |
|---|---|
| H du Me en 18 | 0,70 (s) |
| H de N< | 2,33 (s) |
| H de 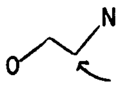 | 2,77 (t) |
| 17 alpha H | 3,62 (t) |
| H de 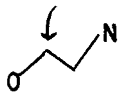 | 4,04 (t) |
| H aromatiques | de 6,50 à 7,22 |

**Exemple 7 : 7 béta-[4-(2-(diméthylamino) éthoxy] phényl] 17 béta-hydroxy estr-4-èn-3-one**

On mélange, sous atmosphère inerte, 480 mg du produit I obtenu à l'exemple 1 (soit le 17 béta-acétoxy 7 béta-[4-[2-(diméthylamino) éthoxy] phényl] estr-4-èn-3-one], 5 cm3 d'éthanol, 1 cm3 de solution méthanolique 2M de potasse, agite à 20¤C pendant 1 heure et 30 minutes, concentre à sec par distillation sous pression réduite, ajoute de l'eau au résidu, extrait à l'acétate d'éthyle, lave à l'eau salée, sèche, concentre à sec par distillation sous pression réduite, chromatographie le résidu (410 mg) sur silice en éluant par un mélange d'acétate d'éthyle et de triéthylamine (9/1), obtient 215 mg de produit brut, dissout ces 215 mg dans 1 cm3 d'acétate d'éthyle bouillant, amorce, refroidit à 0¤C, isole par essorage le précipité formé, le lave, le sèche et obtient 160 mg de produit attendu F = 149¤C.

Recristallisation pour analyse : on dissout 265 mg (deux lots) dans 4 cm3 d'acétate d'éthyle au reflux, filtre, concentre jusqu'à début de cristallisation, refroidit à 0¤C, isole par essorage le précipité formé, le lave à l'acétate d'éthyle glacé, le sèche et obtient 215 mg de produit attendu F = 149¤C.

Spectre de RMN ($CDCl_3$) en ppm :

| | |
|---|---|
| H du Me en 18 | 0,77 (s) |
| H de N< | 2,36 (s) |
| H de 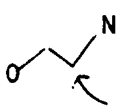 | 2,74 (t) |
| H de 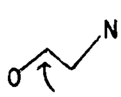 | 4,07 (t) |
| 4H | 5,81 |

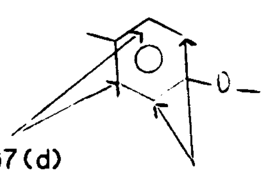

7,07 (d)

6,84 (d)

**Exemple 8 : 7 alpha - [(4-(diméthylamino) phényl] 17 béta-hydroxy estr-4-èn-3-one et 7 beta-[4-(diméthylamino) phényl] 17 béta-hydroxy estr-4-èn-3-one.**

1¤/. Préparation de magnésien :

On mélange, sous atmosphère inerte, 2,1 g de magnésium en copeaux, 5 cm3 de tétrahydrofuranne anhydre, puis après amorçage, à 35¤C, en 45 minutes environ 18 g de 4-bromodiméthylaniline en solution dans 90 cm3 de tétrahydrofuranne, agite encore pendant 1 heure à 20¤C.

Le titre par iodométrie est de 0,65 N.

2¤/. Addition 1-6 :

On refroidit la solution de magnésien obtenue ci-dessus à -40¤C, ajoute en 20 minutes environ une solution de 25 g d'iodure cuivreux, 11,7 g de bromure de lithium dans 100 cm3 de tétrahydrofuranne, agite pendant 15 minutes, ajoute à -50¤C en 15 minutes environ à la suspension épaisse obtenue 17 cm3 d'éthérate de trifluorure de bore, agite pendant 15 minutes, introduit en 5 minutes environ 8 g de 17 béta-hydroxy estra-4,6-dièn 3-one F = 187¤C en solution dans 80 cm3 de tétrahydrofuranne, agite la suspension obtenue à -50¤C pendant 3 heures, puis laisse au repos pendant 16 heures, verse le mélange réactionnel sur 50 cm3 d'acide chlorhydrique au demi, agite 20 minutes à 20¤C, alcalinise par 80 cm3 d'ammoniaque au demi, agite, décante, extrait à l'acétate d'éthyle, lave par une solution aqueuse de chlorure de sodium, sèche, concentre à sec par distillation sous pression réduite, chromatographie le résidu (13 g) sur silice en éluant par un mélange de cyclohexane et d'acétate d'éthyle (1/1), obtient 7,85 g de produitbrut que l'on chromatographie sur silice en éluant par un mélange d'acétonitrile et d'eau (6/4) et obtient :

- 3,38 g de 7 alpha-[4-(diméthylamino) phényl] 17 béta-hydroxy estr-4-èn-3-one F¤ = 190¤C (I) et 1,9 g de 7 béta-[4-diméthylamino) phényl] 17 béta-hydroxy estr-4-èn-3-one (II).

On dissout 830 mg de (I) dans 4 cm3 de chlorure de méthylène, ajoute 8 cm3 d'éther isopropylique, concentre jusqu'à début de cristallisation, amène à 0¤C, isole par essorage le précipité formé, le lave à l'éther isopropylique, le sèche et obtient 715 mg de produit I attendu purifié F = 202¤C.

Constantes du produit (I) (7 alpha).

Spectre de RMN ($CDCl_3$) en ppm :

| | |
|---|---|
| H du Me en 18 | 0,83 (s) |
| H de 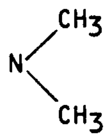 | 2,92 (s) |
| 4 H | 2,92 |
| H aromatiques | 6,66 (d) |
| | 7,06 (d) |

Constantes du produit II (7 béta)

Spectre de RMN ($CDCl_3$) en ppm :

| | |
|---|---|
| H du méthyle en 18 | 0,77 (s) |
| H de 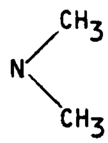 | 2,93 (s) |
| 17 alpha H | 3,53 |
| 4H | 5,82 |
| H aromatiques | 6,72 (d) |
| | 7,15 (d) |

**Exemple 9 : 7 alpha -[4-(diméthylamino) phényl] 17 béta-[diméthyl (1,1-diméthyléthyl) silyloxy] estr-4-èn-3-one**

On dissout à 20¤C, sous atmosphère inerte, 1,36 g de 7 alpha-[4-(diméthylamino) phényl] 17 béta-hydroxy estr-4-èn-3-one obtenu à l'exemple 8 dans 6 cm3 de diméthylformamide, ajoute 630 mg de diméthyl (1,1-diméthyléthyl) chlorosilane, 593 mg d'imidazole, agite à 45¤C pendant 1 heure et 30 minutes, refroidit, verse le mélange réactionnel dans l'eau, extrait à l'acétate d'éthyle, lave à l'eau salée, sèche, concentre à sec par distillation sous pression réduite, chromatographie le résidu (2,15 g) sur silice en éluant par un mélange de cyclohexane et d'acétate d'éthyle (7/3) et obtient 1,54 g de produit attendu.

Spectre de RMN ($CDCl_3$) en ppm :

| | |
|---|---|
| H de Si $Me_2$ | 0,055 (s) |
| H du méthyle en 18 | 0,89 (s) |
| H de tBu | 0,96 (s) |
| H de 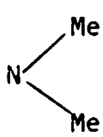 | 3,03 (s) |
| 17 alpha H | 3,5 |
| 4H | 5,97 |
| H aromatiques | 6,72 (d) |
| | 7,12 (d) |

**Exemple 10 : 7 alpha-[4-(diméthylamino) phényl] 17 béta-[diméthyl (1,1-diméthyléthyl) silyloxy] estra 1,3,5(10)-trièn-3-ol**

1¤/. Préparation de l'éther d'énol silylé :

On mélange, sous atmosphère inerte, 0,42 cm3 de diisopropylamine, 8 cm3 de tétrahydrofuranne, ajoute à 0¤C en 10 minutes environ 1,85 cm3 d'une solution 1,65N de n-butyl lithium dans l'hexane, agite pendant 30 minutes, ajoute à -60¤C en 5 minutes environ 394 mg de 7 alpha-[4-(diméthylamino) phényl] 17 béta-[diméthyl (1,1-diméthyléthyl) silyloxy] estra 4-èn 3-one en solution dans 4 cm3 de tétrahydrofuranne, agite pendant 1 heure, ajoute 0,63 cm3 de chlorure de triméthyl silyle, laisse revenir à 20¤C et obtient une

solution.

2/. Aromatisation :

On ajoute dans la solution d'éther d'énol silylé obtenue à l'exemple 9, 340 mg de 2,3-dichloro 5,6-dicyano benzoquinone, 0,5 cm3 d'eau, agite pendant 20 heures à 20¤C, filtre, verse le filtrat sur de la soude, extrait à l'acétate d'éthyle, lave à l'eau salée, sèche, concentre à sec par distillation sous pression réduite, chromatographie le résidu (840 mg sur silice en éluant, par un mélange de toluène et d'acétate d'éthyle (9/1) et obtient 116 mg de produit attendu F = 214¤C.

| Spectre IR (chloroforme) en cm-1 : | |
|---|---|
| OH type phénol | 3 599 cm-1 |
| aromatique | 1 613, 1 582, 1 556, 1 521, 1 501 |

| Spectre de RMN ($CDCl_3$) en ppm : | |
|---|---|
| H de Si $Me_2$ | 0,017 (s) |
| H de 18 Me | 0,77 (s) |
| H de tBu | 0,88 (s) |
| H de $NMe_2$ | 2,9 (s) |
| H aromatiques | 6,56 à 7,28 |

**Exemple 11 : 7 alpha - [4-(diméthylamino) phényl] estra-1,3,5(10)-trièn-3,17 béta-diol**

On dissout à 20¤C, sous atmosphère inerte, 420 mg de 7 alpha-[4-(diméthylamino) phényl] 17 béta-diméthyl (1,1-diméthyléthyl) silyloxy] estra-1,3,5(10)-trièn-3-ol obtenu à l'exemple 10 dans 8 cm3 d'éthanol, ajoute 2 cm3 de solution aqueuse d'acide fluorhydrique à 40%, agite à 20¤C pendant 4 heures et 30 minutes, alcalinise au bicarbonate de sodium, extrait à l'acetate d'éthyle, lave par une solution aqueuse de chlorure de sodium, sèche, concentre à sec par distillation sous pression réduite, dissout le résidu (340 mg, pF = 262¤C) dans 20 cm3 d'acétate d'éthyle au reflux, filtre, concentre jusqu'à cristallisation, amène à 0¤C, agite, isole par essorage le précipité formé, le lave, le sèche à l'acétate d'éthyle et obtient 280 mg de produit attendu F = 262¤C.

| Spectre de RMN ($CDCl_3$)+ DMSO en ppm : | |
|---|---|
| H du Me en 18 | 0,78 (s) |
| H de N $Me_2$ | 2,87 (s) |
| H de 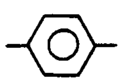 | 6,53 (d)<br>6,91 (d) |
| $H_1$ | 7,13 (d) |
| $H_2$ et $H_4$ | 6,58 à 6,73 |
| 1H mobile | 8,11 |

**Exemple 12 : 17 béta-hydroxy 7 alpha-(4-méthoxyphényl) estr-4-èn-3-one et 17 béta-hydroxy 7 béta-(4-méthoxy phényl) estr-4-èn-3-one**

Dans 50 cm3 d'une solution 0,8M de magnésien du 4-bromoanisole dans le tétrahydrofuranne, on introduit à -40¤C 12,5 g d'iodure cuivreux, 5,5 g de bromure de lithium anhydre en solution dans 50 cm3 de tétrahydrofuranne, agite pendant 15 minutes à -40C, ajoute à -50¤C en 10 minutes environ 8,5 cm3 d'éthérate de trifluorure de bore, agite à -50¤C pendant 15 minutes, introduit 4 g de 17 béta-hydroxy estra-4,6-dièn 3-one F= 187¤C en solution dans 50 cm3 de tétrahydrofuranne, agite la suspension obtenue à -50¤C pendant 3 heures, ajoute 25 cm3 d'acide chlorhydroque au demi et laisse remonter la température ambiante ; on ajoute de l'acétate d'éthyle, agite, décante, lave à l'eau ammoniacale, lave à l'eau salée, sèche, concentre à sec par distillation sous pression réduite, chromatographie le résidu obtenu (6,7 g) sur silice en éluant par un mélange de cyclohexane et d'acétate d'éthyle (1/1), obtient 4,8 g de produit brut, empâte les 4,8 g dans 10 cm3 d'acétate d'éthyle, isole par essorage le précipité, lave à l'acétate d'éthyle, sèche et obtient 2,43 g de 17 béta-hydroxy 7 alpha-(4-méthoxyphényl) estr-4-èn-3-one F=260¤C (produit I)

Les liqueurs mères d'empâtage sont amenées à sec par distillation sous pression réduite et l'on obtient 2,29 g de 17 béta-hydroxy 7 béta-(4-méthoxyphényl) est-4-èn 3-one (produit II).

Constantes du produit I (7 alpha)

| Spectre de RMN ($CDCl_3$) en ppm : | |
|---|---|
| H de 18 Me | 0,83 (s) |
| 17 alpha H | 3,48 (t) |
| H de OMe | 3,78 (s) |
| 4 H | 5,86 |
| H aromatiques | 6,76 (d)<br>7,06 (d) |

Constantes du produit II (7 béta)

| Spectre de RMN ($CDCl_3$) en ppm : | |
|---|---|
| H de 18 Me | 0,77 (s) |
| 17 alpha H | 3,50 (t) |
| H de OMe | 3,78 (s) |
| 4H | 5,79 |
| H aromatiques | 6,79 (d)<br>7,06 (d) |

**Exemple 13 : 17 béta-[diméthyl (1,1-diméthyléthyl) silyloxy] 7 alpha-(4-méthoxyphényl) estr-4-èn 3-one]**

On mélange sous atmosphére inerte 2,3 g de 17 béta-hydroxy 7 alpha-(4-méthoxyphényl) estr-4-én 3-one (produit I), obtenu à l'exemple 12, 12 cm3 de diméthyl formamide, 1,09 g de diméthyl (1,1-diméthyléthyl) chlorosilane, 1,03 g d'imidazole, agite à 40¤C pendant 3 heures et 15 minutes (dissolution), amène à 20¤C, verse le mélange réactionnel dans l'eau, extrait à l'acétate d'éthyle, lave par une solution aqueuse de chlorure de sodium, sèche, concentre à sec par distillation sous pression réduite, chromatographie le résidu (3,84 g) sur silice en éluant par un mélange de cyclohexanne et d'acétate d'éthyle (1/1) et obtient 2,72 g de produit attendu.

| Spectre de RMN ($CDCl_3$) en ppm : | |
|---|---|
| H du $SiMe_2$ | 0,05 (s) |
| H de 18 Me | 0,78 (s) |
| H de tBu | 0,85 (s) |
| 17 alpha H | 3,39 |
| H de OMe | 3,77 (s) |
| 4H | 5,84 |
| H aromatiques | 6,76 (d)<br>7,06 (d) |

**Exemple 14 : 17 béta-[diméthyl (1,1-diméthyléthyl) silyloxy] 7 alpha-(4-méthoxyphényl) estra-1,3,5-(10)-trièn-3-ol**

1¤/. Préparation de l'éther d'énol silylé :

On mélange, sous atmosphère inerte, 0,35 cm3 de diisopropylamine, 7 cm3 de tétrahydrofuranne, ajoute à -5¤C en 5 minutes environ 1,7 cm3 de solution 1,65 N de n-butyl lithium dans l'hexane, agite pendant 30 minutes à -5¤C, ajoute à -60¤C en 10 minutes environ 530 mg de 17 béta-(diméthyl (1,1-diméthyléthyl) silyloxy] 7 alpha-(4-méthoxyphényl) estr-4-èn-3-one obtenu à l'exemple 13, en solution dans 4 cm3 de tétrahydrofuranne, agite à -65¤C pendant 1 heure et 30 minutes, ajoute 0,7 cm3 de chlorure de triméthylsilyle, laisse revenir à 20¤C, agite pendant 1 heure à 20¤C.

2¤/. Déshydrogénation :

On ajoute au mélange réactionnel précédemment obtenu 366 mg de 2,3-dichloro 5,6-dicyano benzoquinone, 0,7 cm3 d'eau, agite pendant 16 heures à 20¤C, verse le mélange réactionnel sur une solution aqueuse 2N de soude extrait à l'acétate d'éthyle, lave par une solution aqueuse de chlorure de sodium, sèche, concentre à sec par distillation sous pression réduite, chromatographie le résidu (880 mg) sur silice en éluant par un mélange de toluène et d'acétate d'éthyle (9/1) et obtient 293 mg de produit attendu.

| Spectre de RMN ($CDCl_3$) en ppm : | |
|---|---|
| H du Si $Me_2$ | 0,017 (s) |
| H de 18 Me | 0,79 (s) |
| H de tBu | 0,91 (s) |
| 17 alpha H | 3,50 |
| H de OMe | 3,78 (s) |
| H aromatiques | 6,7 à 7,27 |

**Exemple 15 : 7 alpha-(4-méthoxyphényl) estra-1,3,5(10)-trièn-3,17-béta-diol**

On dissout à 20¤C, sous atmosphère inerte, 646 mg de 17 béta-[diméthyl(1,1-diméthyléthyl) silyloxy] 7 alpha-(4-méthoxyphényl) estra-1,3,5(10)-trièn 3-ol obtenu à l'exemple 14, dans 50 cm3 d'acétonitrile, ajoute 5 cm3 de solution aqueuse à 40 % d'acide fluorhydrique, il y a formation d'une gomme que l'on dissout par addition de 40 cm3 d'acétonitrile, abandonne à 20¤C pendant 16 heures, concentre par distillation sous pression réduite, neutralise au bicarbonate de sodium, extrait à l'acétate d'éthyle, lave par une solution aqueuse de chlorure de sodium, sèche, concentre à sec par distillation sous pression réduite, chromatographie le résidu (1,02 g) sur silice en éluant par un mélange de cyclohexane et d'acétate déthyle (1/1) et obtient 370 mg de produit attendu F = 190¤C, puis 227¤C.

Cristallisation pour analyse :

On dissout les 370 mg obtenus précédemment dans 12 cm3 d'acétate d'éthyle au reflux, filtre, concentre jusqu'à cristallisation, amène à 0¤C, agite, isole par essorage le précipité formé, le lave à l'acétate d'éthyle, le sèche et obtient 300 mg de produit attendu purifié F = 190¤C, puis 228¤C.

| Spectre de RMN ($CDCl_3$) en ppm : | |
|---|---|
| H de 18 Me | 0,8 (s) |
| H de OMe | 3,74 (s) |
| H mobile | 4,71 |
| H aromatiques | de 6,61 à 7,22 |

**Exemple 16 : 17 béta-hydroxy 7 alpha-[4-(méthylthio) phényl] estr-4-èn-3-one et 17 béta-hdyroxy 7 béta-[4-(méthylthio) phényl] estr-4-èn-3-one**

1¤/. Préparation du magnésien :

On mélange, sous atmosphère inerte, à 20¤C, 2 g de magnésium en copeaux, 2 cm3 de tétrahydrofuranne, introduit, aprés amorçage par le dibromométhane en 1 heure environ 15 g de 4-bromothioanisole en solution dans 45 cm3 de tétrahydrofuranne, durant l'introduction la température se maintient à 50¤C, on agite encore 1 heure à 50¤C, puis titre par iodométrie. Le titre du magnésien est de 1,1M.

2¤/. Addition 1,6 :

On mélange, sous atmosphère inerte, 50 cm3 de magnésien 1,1M obtenu ci-dessus, 15 g d'iodure cuivreux, 7 g de bromure de lithium en solution dans 40 cm3 de tétrahydrofuranne, agite pendant 15 minutes, introduit à -50¤C en 5 minutes environ, 10 cm3 d'éthérate de trifluorure de bore, agite pendant 15 minutes à -50¤C, introduit en 15 minutes environ 4,9 g de 17 béta-hydroxy estra 4,6-dién 3-one F = 187¤C, en solution dans 50 cm3 de tétrahydrofuranne, agite la suspension obtenue pendant 1 heure, verse le mélange réactionnel dans un mélange de solution aqueuse de chlorure d'ammonium et de glace, agite, ajoute une solution aqueuse 5M d'ammoniaque, décante, extrait à l'acétate d'éthyle, lave par une solution aqueuse saturée de chlorure de sodium, sèche, concentre à sec par distillation sous pression réduite, chromatographie le résidu (14,5 g) sur silice, sous pression d'azote, en éluant par un mélange de cyclohexane et d'acétate d'éthyle (1/1) et obtient 2,89 g de mélange d'épimers F ≃ 220¤C.

Cristallisation :

On dissout les 2,89 g obtenus ci-dessus dans 35 cm3 de chlorure de méthylène, à l'ébullition, ajoute 35 cm3 d'éther isopropylique, concentre jusqu'à cristallisation, refroidit à 0¤C, agite, isole par essorage le précipité formé, le lave à l'éther isopropylique, le sèche et obtient 1,65 g de 17 béta-hydroxy 7 alpha-[4-(méthylthio) phényl] estr-4-èn-3-one (produit I).

On dissout 400 mg du produit I dans 20 cm3 de méthanol bouillant, filtre, concentre jusqu'à début de cristallisation, glace, isole par essorage le précipité formé, le lave, le sèche et obtient 330 mg F = 244¤C.
Constants du produit I

| Spectre de RMN ($CDCl_3$) en ppm : | |
|---|---|
| H du Méthyle en 18 | 0,82 (s) |
| H de $SCH_3$ | 2,45 (s) |
| 17 alpha H | 3,49 (t) |
| $H_4$ | 5,87 |
| H aromatiques | 7,0 à 7,2 |

On amène à sec les liqueurs-mères de cristallisation du produit I et obtient 1,24 g de produit II contenant 3/4 d'isomère 7 béta, soit de 17 béta-hydroxy 7 béta-[4-(méthylthio] phényl] estr-4-èn 3-one.
Constante du produit II.

| Spectre de RMN ($CDCl_3$) en ppm : | |
|---|---|
| H du méthyle en 18 | 0,77 (s) |
| H de $SCH_3$ | 2,47 (s) |
| 17 alpha H | 3,51 (s) |
| $H_4$ | 5,80 |
| H aromatiques | 7,0 à 7,2 |

**Exemple 17 : 17 béta-[diméthyl (1,1-diméthyléthyl) silyloxy] 7 alpha-[4-(méthylthio) phényl] estr-4-èn-3-one**

On met en suspension, sous atmosphère inerte, 0,9 g de 17 béta-hydroxy 7 alpha-[4-(méthylthio) phényl] estr-4-èn 3-one (produit obtenu à l'exemple 16), 500 mg de diméthyl (1,1-diméthyléthyle) chlorosilane, 476 mg d'imidazole, dans 5 cm3 de diméthylformamide, agite à 50¤C pendant 1 heure et 25 minutes, on note une dissolution rapide, amène à 20¤C, verse le mélange réactionnel dans l'eau, extrait à l'acétate d'éthyle, lave par une solution aqueuse saturée de chlorure de sodium, sèche, concentre à sec par distillation sous pression réduite, chromatographie le résidu (1,39 g) sur silice en éluant par un mélange de cyclohexane et d'acétate d'éthyle (7/3) et obtient 916 mg de produit attendu.

| Spectre de RMN ($CDCl_3$) en ppm : | |
|---|---|
| H du Si $Me_2$ | 0,05 (s) |
| H de 18 Me | 0,79 (s) |
| H de tBu | 0,84 (s) |
| H de SMe | 2,51 (s) |
| $H_4$ | 5,86 |
| H aromatiques | 7,08 |

**Exemple 18 : 17 béta-[diméthyl (1,1-diméthyléthyl) silyloxy] 7 alpha-14-(méthylthio) phényl] estra-1,3,5(10)-trièn-3-ol**

On mélange, sous atmosphère inerte, 0,49 cm3 de diisopropylamine, 10 cm3 de tétrahydrofuranne, ajoute à 0¤C en 5 minutes environ 2,2 cm3 de suspension de n-butyl lithium 1,65 N dans l'hexane, agite pendant 30 minutes, introduit à -70¤C, en 20 minutes environ 890 mg de 17 béta -[diméthyl (1,1-diméthyléthyl) silyloxy] 7 alpha-[4-(méthylthio) phényl] estr-4-èn-3-one obtenu à l'exemple 17, en solution dans 6 cm3 de tétrahydrofuranne, agite pendant 30 minutes, introduit 1,05 cm3 de chlorure de triméthylsilyle, laisse revenir à 20C, ajoute 600 mg de 2,3-dichloro 5,6-dicyano benzoquinone, 1 cm3 d'eau, laisse au repos à 20¤C pendant 16 heures, verse le mélange réactionnel dans 50 cm3 de solution aqueuse 2 N de soude, extrait à l'acétate d'éthyle, lave par une solution aqueuse saturée de chlorure de sodium, sèche, concentre à sec par distillation sous pression réduite, chromatographie le résidu (1,3 g) sur silice en éluant avec un mélange de toluène et d'acétate d'éthyle (95/5) et obtient 187 mg de produit attendu.

| Spectre IR (chloroforme) en $cm^{-1}$ : | |
|---|---|
| OH | 3 610 |

**Exemple 19 : 7 alpha-[4-(méthylthio) phényl] estra-1,3,5(10)-trièn-3,17 béta-diol**

On dissout, sous atmosphère inerte, 185 mg de 17 béta-[diméthyl (1,1-diméthyléthyl) silyloxy] 7 alpha -[4-(méthylthio) phényl] estra-1,3,5 (10)-trién 3-ol obtenu à l'exemple 18, dans 6 cm3 d'éthanol, ajoute 0,9 cm3 d'acide fluorhydrique à 40%, agite pendant 3 heures à 20¤C, note l'apparition d'un précipité, abandonne pendant 16 heures au repos, verse le mélange réactionnel dans une solution aqueuse de bicarbonate de sodium, agite, isole par essorage le précipité formé, le lave à l'eau, le sèche, obtient 260 mg de produit brut, y ajoute 10 cm3 d'acétate d'éthyle bouillant, élimine par filtration l'insoluble résiduel,

concentre le filtrat à petit volume, amorce, refroidit à 0¤C, agite pendant 1 heure à 0¤C, isole par essorage le précipité formé, le lave, le sèche et obtient 94 mg de produit attendu F = 186¤C.

| Spectre de RMN ($CDCl_3$) en ppm : | |
|---|---|
| H du 18 Me | 0,80 (s) |
| H de SMe | 2,42 (s) |
| $H_{17}$ alpha | 3,56 |
| H de OH | 4,85 |
| H aromatiques | 6,63 à 7,16 |

**Exemple 20 : 7alpha [4-(diméthylamino) phényl] 17béta-hydroxy 19-nor 17alpha-pregn-4-èn-20-yn-3-one**

**Stade A :** (1,2-éthanediyl) acétal cyclique de 7alpha-[4-(diméthylamino) phényl] 17béta-hydroxy estr-5(10)-èn-3-one et 3,3-(1,2-éthanediyl) acétal cyclique de 7alpha [4-(diméthylamino) phényl] 17béta-hydroxy estr-5-èn-3-one.

On chauffe à 75/80¤C sous atmosphère inerte pendant 2 heures et quart, 4 g de produit préparé comme à l'exemple 8, dans 16 cm3 d'éthylène glycol et 8 cm3 d'orthoformiate d'éthyle en présence de 4 g d'acide paratoluènesulfonique dihydraté. On laisse revenir à température ambiante, alcalinise à l'aide de bicarbonate de sodium, extrait à l'acétate d'éthyle, lave à l'eau salée, sèche et élimine les solvants sous pression réfuite et obtient 7,49 g de produit brut que l'on chromatographie sur silice (éluant : éther de pétrole (eb. 40¤-70¤C)-acétate d'éthyle 1-1) et obtient 3,07 g de produit delta 5(10) et 0,470 g de produit delta 5, F = 220¤C attendus.

**Stade B :** 3,3-(1,2-éthanediyl) acétal cyclique de 7alpha-[4-(diméthylamino) phényl] estr-5(10)-èn-3,17-dione et 3,3-(1,2-éthanediyl) acétal cyclique de 7alpha-[4-(diméthylamino) phényl] estr-5-èn-3,17-dione.

On chauffe au reflux sous atmosphère inerte 2,63 g du mélange obtenu comme au stade A dans 50 cm3 de toluène avec 1,2 g d'isopropylate d'aluminium puis introduit en 3 heures 4 cm3 de cyclohexanone dans 20 cm3 de toluène tout en distillant. On laisse revenir à température ambiante, lave à l'eau salée, extrait à l'acétate d'éthyle, sèche et élimine les solvants sous pression réduite. On obtient 4,76 g de produit brut que l'on chromatographie sur silice (éluant : éther de pétrole (eb. 40¤-70¤C)-acétate d'éthyle-triéthylamine 60-40-0,1) et recueille 2,05 g de produit delta 5(10), F = 208¤C et 0,14 g de produit delta 5, F = 210¤C attendus.

| Produit delta 5(10) : Analyse $C_{28}H_{37}NO_3$ : 435,61 | | | |
|---|---|---|---|
| Calculé : | C% 77,20 | H% 8,56 | N% 3,22 |
| Trouvé : | 77,3 | 8,7 | 2,9 |

**Stade C :** (1,2-éthanediyl) acétal cyclique de 7alpha [4-(diméthylamino) phényl] 17béta-hydroxy 19-nor 17alpha-pregn-5(10)-èn-20-yn-3-one.

On fait barboter pendant 1 heure de l'acétylène dans 17 cm3 de tertbutylate de potassium en solution 0,955N dans le tétrahydrofuranne, 3,4 cm3 d'hexaméthylphosphotriamide et 17 cm3 de tétrahydrofuranne refroidi à +4¤C. On ajoute 1,15 g du produit delta 5(10) préparé au stade précédent en solution dans 20 cm3 de tétrahydrofuranne. On laisse revenir à température ambiante, agite pendant 2 heures, verse dans une solution de chlorure d'ammonium, extrait à l'acétate d'éthyle, lave à l'eau salée, sèche et élimine les solvants sous pression réduite à 50¤C. On obtient 3,1 g de produit brut que l'on chromatographie sur silice (éluant : toluène-acétate d'éthyle-triéthylamine 80-20-0,1) et recueille 1,35 g de produit attendu.

| Spectre IR ($CHCl_3$) | |
|---|---|
| OH<br>-C=CH<br>aromatique | : 3601 $cm^1$<br>: 3306 $cm^1$<br>: 1614 - 1556 - 1520 $cm^{-1}$ |

**Stade D :** 7alpha-[4-(diméthylamino) phényl] 17béta-hydroxy 19-nor 17alpha-pregn-4-èn-20-yn-3-one.

On chauffe à 35¤-40¤C pendant 3 heures sous atmosphère inerte 1,3 g de produit obtenu au stade C dans 13 cm3 de méthanol et 2,6 cm3 d'acide chlorhydrique 2N. On élimine le méthanol sous pression réduite, alcalinise à l'aide de bicarbonate de sodium, extrait à l'acétate d'éthyle, lave à l'eau salée, sèche et amène à sec sous pression réduite. On recueille 1,17 g de produit brut que l'on chromatographie sur silice (éluant : éther de pétrole (eb. 40¤-70¤C)-acétate d'éthyle 1-1) et obtient 0,81 g de produit attendu. F = 210¤C.

| Analyse : $C_{28}H_{35}NO_2$ : 417,60 | | | |
|---|---|---|---|
| Calculé :<br>Trouvé : | C% 80,53<br>80,6 | H% 8,45<br>8,5 | N% 3,35<br>3,2 |

| Spectre IR ($CHCl_3$) | |
|---|---|
| OH<br>-C=CH<br>cétone conjuguée<br>aromatique | : 3601 $cm^{-1}$<br>: 3306 $cm^{-1}$<br>: 1660 $cm^{-1}$<br>: 1614 - 1556 - 1520 $cm^{-1}$ |

**Exemple 21 : 7alpha-[4-(diméthylamino) phényl] 17béta-hydroxy 17alpha-(1-propynyl) estr-4-èn-3-one.**

**Stade A :** (1,2-éthanediyl) acétal cyclique de 7alpha [4-(diméthylamino) phényl] 17béta-hydroxy 17alpha-(1-propynyl) estr-5(10)-èn--3-one.

On opère comme au stade C de l'exemple 20 à partir de 0,875 g de produit préparé comme au stade B de l'exemple 20 et en remplaçant l'acétylène par le méthylacétyléne. Après la chromatographie de 1,5 g de produit brut obtenu, on recueille le produit attendu.

| Spectre IR ($CHCl_3$) | |
|---|---|
| OH<br>-C=CH<br>aromatique | : 3603 $cm^{-1}$<br>: 2240 $cm^{-1}$<br>: 1613 - 1560 - 1519 $cm^{-1}$ |

**Stade B :** 7alpha-[4-(diméthylamino) phényl] 17béta-hydroxy 17alpha-(1-propynyl) estr-4-èn-3-one.

On opère comme au stade D de l'exemple 20 à partir de 1,05 g de produit obtenu au stade A ci-dessus. On obtient 0,94 g de produit brut q l'on chromatographie sur silice (éluant : éther de pétrole (eb. 40-70¤C acétate d'éthyle 4-6) puis dissout le ésidu obtenu dans le chlorure de méthylène bouillant, ajoute 9 cm3 d'éther isopropylique, concentre partiellement, glace, essore, sèche à 50¤C sous pression réduite et recueille 0,49 g de produit attendu. F = 217¤C.

| Analyse : $C_{29}H_{37}NO_2$ 431,63 | | | |
|---|---|---|---|
| Calculé : | C% 80,70 | H% 8,64 | N% 3,24 |
| Trouvé : | 80,6 | 8,9 | 3,1 |

| Spectre IR ($CHCl_3$) | |
|---|---|
| OH | : 3602 $cm^{-1}$ |
| -C≡CH | : 2235 $cm^{-1}$ |
| acétone conjuguée | : 1660 - 883 $cm^{-1}$ |
| aromatique | : 1613 - 1522 $cm^{-1}$ |

**Exemple 22 : 7alpha-[4-(diméthylamino) phényl] 17béta-hydroxy estr-4-èn-3-one et 7béta-[4-(diméthylamino) phényl] 17béta-hydroxy estr-4-èn-3-one.**

**Stade A :** 17béta-[diméthyl (1,1-diméthyléthyl) silyloxy] estra-4,6-dièn-3-one.

On mélange à température ambiante sous atmosphère inerte 74,55 g d 17béta-hydroxy estra-4,6-dièn-3-one, 4,6 g d'imidazole et 49 g de chloru de terbutyldiméthylsilyle dans 210 cm3 de diméthylformamide. On chauffe 50¤C pendant 2 heures et quart, ramène à température ambiante, verse da l'eau, essore, sèche sous pression réduite à 50¤C et récupère 110 g de produit brut que l'on purifie par chromatographie sur silice (éluant : cyclohexane-acétate d'éthyle 7-3) et obtient 92 g de produit attendu. F = 110¤C.

**Stade B :** 7-[4-(diméthylamino) phényl] 17béta-[diméthyl (1,1-diméthyl éthyl) silyloxy] estr-4-èn-3-one.

On refroidit à 0¤/+5¤C sous atmosphère inerte, 500 cm3 de bromure 4-(diméthylamino) phényl magnésium ensolution 0,9N dans le tétrahydrofuranne, ajoute 9 g de CuBr, $Me_2S$, agite 15 minutes, refroidit à -65¤C ajoute 20 cm3 de chlorure de triméthylsilyle, puis en 40 minutes 38,7 g produit préparé au stade A en solution dans 390 cm3 de tétrahydrofuranne et 43 cm3 d'hexaméthylphsophotriamide. On agite 45 minutes, ajoute 350 cm3 d'acide chlorhydrique 2N, laisse revenir à température ambiante, agite 1 heure 45 minutes, alcalinise avec 70 cm3 d'ammoniaque concentré, décante, lave à l'eau salée, extrait à l'acétate d'éthyle, sèche, élimine les solvants sous pression réduite et recueille 114 g de produit brut que l'on purifie par chromatographie sur silice (éluant : éther de pétrole (eb. 40¤-70¤C)-acétate d'éthyle 8-2) et obtient 31 g de produit attendu.

**Stade C :** 7alpha-[4-(diméthylamino) phényl] 17béta-hydroxy estr-4-èn-3-one et 7béta-[4-(diméthylamino) phényl] 17béta-hydroxy estr-4-èn-3-one.

On ajoute 90 cm3 d'acide chlorhydrique 2N dans 31 g du produit obtenu au stade B en suspension dans 300 cm3 de méthanol, agite 1 heure à température ambiante, verse dans 300 cm3 de solution aqueuse de bicarbonate de sodium, extrait à l'acétate d'éthyle, lave à l'eau salée, sèche et élimine les solvants sous pression réduite. On reprend le résidu dans 20 cm3 d'acétonitrile, amorce la cristallisation, glace, essore et sèche les cristaux à 50¤C sous pression réduite. On recueille 3,1 g de produit attendu (isomère 7alpha) identique au produit obtenu à l'exemple 8. F = 200¤C.

On chromatographie 26,3 g provenant des liqueurs mères sur silice (éluant : éther de pétrole (eb. 40¤-70¤C)-acétate d'éthyle 4-6), cristallise le résidu dans l'acétonitrile et obient 4,45 g d'isomère 7alpha. Une chromatographie sur silice de 1,34 g de résidu provenant des liqueurs mères (éluant : acétonitrile-eau 6-4) permet d'obtenir 170 mg d'isomère 7alpha, 160 mg d'isomère 7béta et 450 mg de mélange d'isomère.

| Analyse de l'isomère 7 alpha : $C_{26}H_{35}O_2N$ : 379,57 | | | |
|---|---|---|---|
| Calculé : | C% 79,35 | H% 8,96 | N% 3,56 |
| Trouvé : | 79,2 | 9,2 | 3,5 |

**Exemple 23 : Exemple de composition pharmaceutique**

On a préparé des comprimés répondant à la formule suivante :

| | |
|---|---|
| - produit de l'exemple 1<br>- excipient q.s. pour un comprimé terminé à détail de l'excipient (talc, amidon, stéarate de magnésium). | 100 µg<br>100 mg |

**ETUDE PHARMACOLOGIOUE DES PRODUITS DE L'INVENTION**

. Activité anti-proliférative des produits sur la croissance de cellules tumorales mammaires MCF-7.

Description du test :

a/. culture cellulaire :

Les lignées MCF-7 sont maintenues en culture en milieu SVF(1) à 37¤C en atmosphère humide contenant 5% $CO_2$. Les cellules à subconfluence sont récoltées par trypsination (trypsine 0,05%, EDTA 0,02 %) puis rincées par centrifugation douce. Un échantillon des cellules en suspension est compté sur cellule de Malassez.

b/. étude de la croissance :

Les cellules resuspendues dans le milieu SVF sont ensemencées à raison de 30.000 cellules par puits dans des plaques multipuits (24 puits de 2,5 cm2). Vingt quatre heures après l'ensemencement (J0), le produit à tester est ajouté au milieu en solution éthanolique (concentration finale en éthanol : 0,1%), à la concentration de $10^{-5}$M, les puits contrôles recevant la même concentration en éthanol. Les milieux sont renouvelés toutes les 48 Heures. En fin d'expérience (J6), le milieu est aspiré et les cellules sont immédiatement fixées par 150 ul de méthanol afin de doser l'ADN.
L'activité anti-proliférative des produits est évaluée par leur capacité à inhiber l'augmentation d'ADN.

c/. Dosage de l'ADN :

L'ADN est dosé par une méthode fluorimétrique utilisant le DABA (Acide 3,5 diaminobenzoïque) (2) 150 ul de DABA sont ajoutés dans chaque puits ; les plaques sont alors incubées 45 mn à 56¤ C, puis 1,5 ml d'HCl 1N sont ajoutés. La fluorescence est mesurée à l'aide d'un fluorimètre (longueur excitatrice : 400 nm, longueur d'onde d'émission : 500 nm).
La quantité d'ADN par puits est évaluée par rapport à une gamme étalon obtenue en traitant dans les mêmes conditions un standard d'ADN de thymus de veau.

Résultats :

La concentration en nM qui inhibe de 50 % la croissance des cellules $MCF_7$ (CI50) a été déterminée de la manière indiquée ci-dessus pour les produits des exemples 3, 4 et 6. On a obtenu les résultats suivants :

Produit de l'exemple 3 : CI50 = 0,1 nM
Produit de l'exemple 4 : CI50 = 100 nM
Produit de l'exemple 6 : CI50 = 10 nM

(1) Le milieu de culture sérum de veau foetal (SVF) est préparé comme suit :
Milieu MEM (minimal Essential Medium) auquel sont ajoutés :
- acides aminés non essentiels (GIBCO),
- péni-strepto (penicilline 100U/ml, streptomycine 0,1 mg/ml),
- fungizone 0,1 %,
- insuline (5ng/ml),
- sérum de veau foetal (4% concentration finale).

(2) Puzas et Goodman, Analytical Biochemistry, Vol. 86, pp. 50, 1978.

**Revendications**

**Revendications pour les Etats contractants suivants : AT, BE, CH, DE, FR, GB, IT, LI, LU, NL, SE**

**1.** Les produits de formule générale (I) :

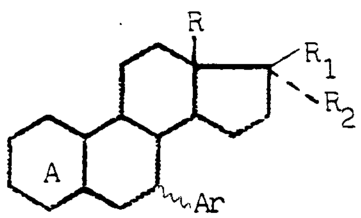

(I)

dans laquelle le cycle A présente l'une des structures suivantes :

a/. soit A représente le groupement :

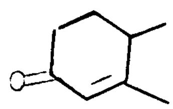

b/. soit A représente le groupement :

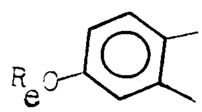

dans lequel Re représente un atome d'hydrogène, un radical alkyle ayant de 1 à 6 atomes de carbone éventuellement substitué ou un radical acyle,

R représente un radical méthyle ou éthyle,

$R_1$ représente un radical hydroxyle éventuellement protégé ou acylé par un radical acyle choisi parmi les radicaux acétyle, chloroacétyle ou trifluoroacétyle, ou $R_1$ représente un radical alcoxyle,

$R_2$ représente un atome d'hydrogène, un radical alkyle, alkényle ou alkynyle ayant au plus 8 atomes de carbone ou un radical aryle ou aralkyle ayant au plus 15 atomes de carbone, chacun de ces radicaux alkyle, alkényle, alkynyle, aryle ou aralkyle étant éventuellement substitué, par un ou plusieurs substituants identiques ou différents choisis parmi les radicaux :

- halogène,
- alkoxy,
- alkylthio,
- amino, alkylamino, dialkylamino, chacun des radicaux dialkylamino étant éventuellement sous forme oxydée,
- aminoalkyl,
- dialkylaminoalkyle,
- dialkylaminoalkyloxy,
- hydroxyle, acétoxy, un radical de formule

$$-O-\underset{\underset{O}{\parallel}}{C}-(CH_2)nCO_2H$$

  dans lequel n = 2 à 5,
- acyle,

- carboxy libre ou estérifié,
- cyano,
- trifluorométhyle,
- aryle ou aralkyle,

les radicaux aryle et aralkyle, que peut représenter $R_2$ pouvant être de plus substitués par un radical alkyle,
soit $R_1$ et $R_2$ forment ensemble un radical choisi parmi les radicaux :

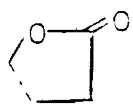 ; 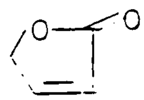 ; 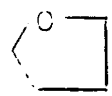 ; 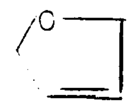

Ar représente un radical aryle ayant 5 ou 6 chaînons carbocyclique ou hétérocyclique, choisi parmi les radicaux thiényle, furyle, thiazolyle, pyrrolyle, oxazolyle, imidazolyle, pyrazolyle, triazolyle (1,2,3 ou 1,2,4) tétrazolyle, isothiazolyle, isoxazolyle, thiadiazolyle, phényle, pyridinyle, pyridazinyle, pyrimidinyle, pyrazinyle, pipéridinyle,
ces radicaux étant éventuellement substitués par un ou plusieurs substituants identiques ou différents choisis parmi les radicaux :

- halogène,
- alkyle,
- alkoxy,
- alkylthio,
- amino, alkylamino, dialkylamino, chacun des radicaux dialkylamino étant éventuellement sous forme oxydée,
- aminoalkyl,
- dialkylaminoalkyle,
- dialkylaminoalkyloxy,
- hydroxyle, acétoxy, un radical de formule

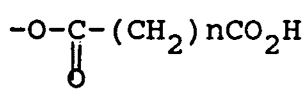

  dans lequel n = 2 à 5,
- acyle,
- carboxy libre ou estérifié,
- cyano,
- trifluorométhyle,
- aryle ou aralkyle,

ainsi que les sels d'addition des produits de formule (I) avec les acides et les bases.

**2.** Les produits de formule (I) telle que définie à la revendication 1, dans laquelle le cycle A représente le groupement :

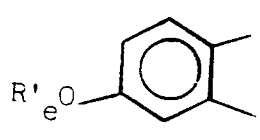

dans lequel $R'_e$ représente un atome d'hydrogène ou un radical alkyle ayant de 1 à 4 atomes de carbone.

**3.** Les produits de formule (I) telle que définie à l'une des revendications 1 ou 2, dans laquelle Ar

représente :

a/. soit un radical phényle éventuellement substitué par un ou plusieurs substituants choisis parmi les radicaux halogène, alkyle, alkoxy ou alkylthio ayant de 1 à 4 atomes de carbone, amino, alkylamino, dialkylamino, dialkylamino alkoxyle, hydroxyle, acyle, carboxy libre estérifié ou salifié, cyano, trifluorométhyle, phényle ou benzyle eux-mêmes éventuellement substitués par un ou plusieurs radicaux alkyles ayant de 1 à 4 atomes de carbone,

b/. soit un radical hétérocyclique choisi parmi les radicaux thiényle, furyle, thiazolyle, isothiazolyle, oxazolyle, isoxazolyle, thiadiazolyle, pyridinyle, pipéridinyle.

**4.** Les produits de formule (I) telle que définie à l'une des revendications 1 à 3, dans laquelle $R_1$ représente un radical hydroxyle éventuellement protégé ou acylé, $R_2$ représente un radical alkyle, alkényle ou alkynyle, chacun de ces radicaux étant éventuellement substitué par un radical choisi parmi les halogènes, les radicaux hydroxyle, carboxy éventuellement estérifié ou salifié ou cyano.

**5.** Les produits de formule générale (I), telle que définie à l'une des revendications 1 à 4, dont les noms suivent :

- Le 7alpha-[4-[2-(diméthylamino) éthoxy] phényl] estra-1,3,5(10)-trièn-3,17béta-diol.
- La 7alpha-[4-[2-(diméthylamino) éthoxy] phényl] 17béta-hydroxy estr-4-èn-3-one.
- Le 7béta-[4-[2-(diméthylamino) éthoxy] phényl] estra-1,3,5(10)-trièn-3,17béta-diol.
- La 7béta-[4-(diméthylamino) éthoxy] phényl] 17béta-hydroxy estr-4-èn-3-one.
- Le 7alpha -[4-[2-(diméthylamino) phényl] estra-1,3,5(10)-trièn-3,17 béta-diol.
- Le 7alpha -(4-méthoxyphényl) estra-1,3,5(10)-trièn-3,17béta-diol.
- La 17béta-hydroxy 7alpha-[4-(méthylthio) phényl] estr-4-èn-3-one.
- Le 7alpha-[4-(méthylthio) phényl] estra-1,3,5(10)-trièn-3,17béta-diol.

**6.** Procédé de préparation des produits de formule générale (I) telle que définie à la revendication 1, caractérisé en ce l'on soumet un produit de formule (II) :

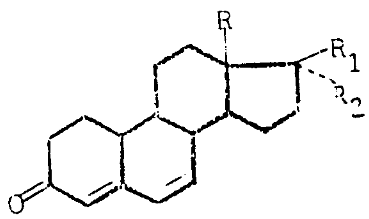

(II)

dans laquelle R, $R_1$ et $R_2$ ont la signification indiquée à la revendication 1 d'abord à l'action, éventuellement en présence d'un acide de Lewis,

- soit d'un produit de formule ($III_a$) :

  Ar-Mg-Hal ($III_a$)

  dans laquelle Ar a la signification indiquée à la revendication 1 et Hal représente un atome d'halogène en présence d'un sel cuivreux :

- soit d'un produit de formule ($III_b$) :

  $Ar_2CuLi$ ($III_b$)

- soit d'un produit de formule ($III_c$) :

  $Ar_2CuCNLi_2$ ($III_c$)

pour obtenir un produit de formule ($I_A$) :

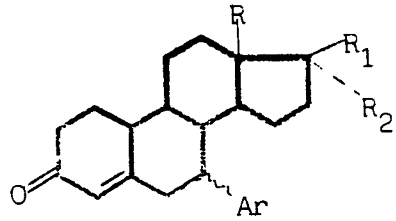

$(I_A)$

éventuellement sous forme d'un mélange d'isomères 7 alpha et 7 béta que l'on sépare, si désiré et produit de formule $I_A$ correspondant à un produit de formule (I) dans laquelle le cycle A représente le groupement :

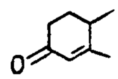

que l'on traite, si désiré par un agent d'aromatisation pour obtenir un produit de formule

$(I_{B_1})$ :

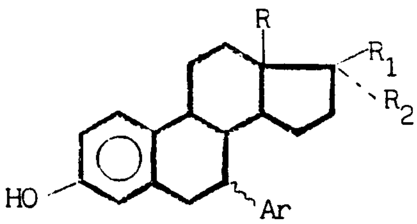

$(I_{B_1})$

dans laquelle Ar, R, $R_1$ et $R_2$ ont la signification indiquée à la revendication 1 et correspondant à un produit de formule I dans laquelle le cycle A représente le groupement :

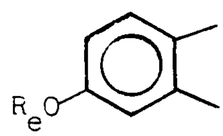

Re représentant un atome d'hydrogène et produit de formule

$(I_{B_1})$

que l'on traite si désiré par un réactif d'alkylation pour obtenir un produit de formule

$(I_{B_2})$ :

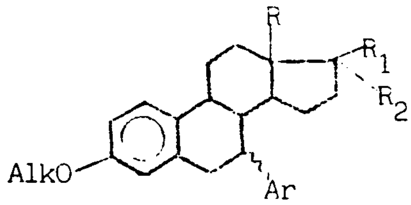

$(I_{B_2})$

dans laquelle Alk représente un radical alkyle ayant de 1 à 6 atome de carbone éventuellement substitué et correspondant à un produit de formule (I) dans lequel le cycle A représente le groupement :

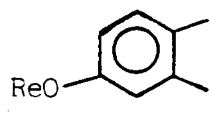

Re représentant un radical alkyle éventuellement substitué, produits de formules $(I_A)$,

$(I_{B_1})$ ou $(I_{B_2})$

que, selon la valeur des substituants $R_2$ et Ar l'on soumet si désiré à l'action d'une base ou d'un acide pour obtenir les sels correspondants.

**7.** Procédé selon la revendication 6, caractérisé en ce que l'on soumet un produit de formule $(I_C)$ :

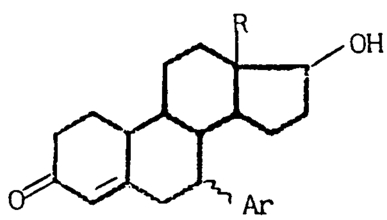

$(I_C)$

dans laquelle R et Ar ont la signification indiquée à la revendication 1, , correspondant à un produit de formule $(I_A)$ dans laquelle $R_1$ représente un radical hydroxyle et $R_2$ représente un atome d'hydrogène à l'action d'un réactif de blocage de la fonction 3-céto par exemple-un éther d'énol ou un cétal- pour obtenir unproduit de formule (IV) :

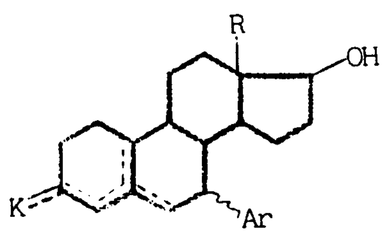

(IV)

dans laquelle K et les traits pointilles représentent une fonction cétonique protégée, de préférence un produit de formules $(IV_A)$ ou $(IV_B)$ :

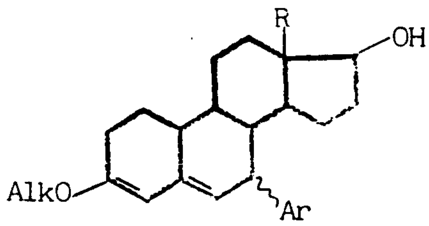

(IV$_A$)

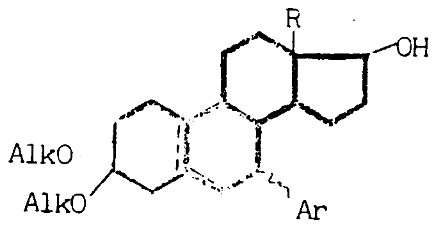

(IV$_B$)

dans laquelle Alk représente de préférence un radical akyle et les traits pointillés représentent une double liaison en 5(6) ou 5(10), produit de formule (IV) que l'on traite par un agent d'oxydation pour obtenir un produit de formule (V) :

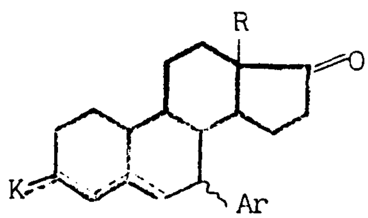

(V)

produit sur lequel on fait agir soit un dérivé organo métallique dérivé d'un radical $R_2$ représentant un radical alkyle, alkényle, alkynyle ayant au plus 8 atomes de carbone ou un radical aryle ou aralkyle ayant au plus 15 atomes de carbone, chacun de ces radicaux alkyle, alkényle, alkynyle, aryle ou aralkyle étant éventuellement substitué, soit d'abord un dérivé organométallique dérivé du réactif de formule $-C{\equiv}C-CH_2OH$ dans lequel la fonction hydroxyle est éventuellement protégée, puis dans un ordre quelconque si désiré à un réactif de déprotection et à un réactif de réduction totale ou partielle, puis à un réactif de cyclisation ou d'oxydation, soit d'abord à un réactif de formule

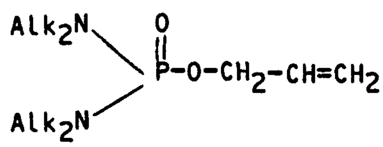

en présence d'une base forte pour obtenir respectivement les produits de formule (VI$_A$) :

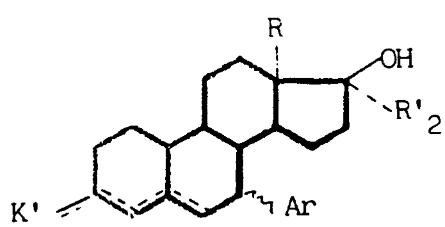

(VI$_A$)

dans laquelle $R'_2$ a les valeurs de $R_2$ à l'exception de la valeur hydrogène et K' et les traits pointillés représentent une fonction 3-céto delta 4 éventuellement protégée et les produits de formule (VI$_B$) :

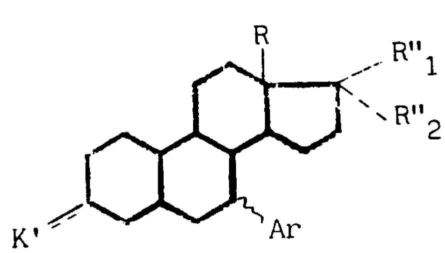

(VI$_B$)

dans laquelle $R''_1$ et $R''_2$ représentent ensemble un radical :

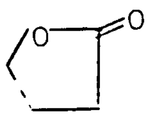 ; 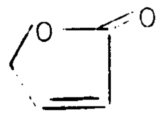 ; 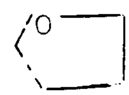 ou 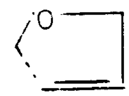

produits de formules ($VI_A$) et ($VI_B$) que l'on soumet le cas échéant à un réactif de déprotection lorsque K'représente un groupement protecteur de la fonction 3-céto delta-4 pour obtenir les produits de formule ($I_D$) :

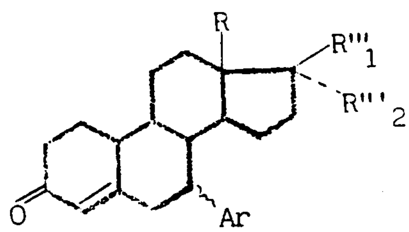

($I_D$)

dans laquelle $R'''_1$ et $R'''_2$ ont les valeurs indiquées ci-dessus pour $R_1$ et R2 à l'exception de la valeur $R_2$ = hydrogène, produits de formule ($I_D$) que l'on peut le cas échéant, lorsque $R'''_1$ représente un radical hydroxyle, soumettre à l'action d'un réactif de protection, d'acylation ou d'alkylation pour obtenir un produit ($I'_D$) correspondant dans lequel la fonction 17béta-OH est protégée, acylée ou alkylée, produit de formules ($I_D$ et ($I'_D$) correspondant à des produits de formule ($I_A$), que l'on peut le cas échéant traiter comme indiqué à la revendication 1.

**8.** A titre de médicaments, les produits de formule générale (I) telle que définie à la revendication 1, ou leurs sels pharmaceutiquement acceptables.

**9.** A titre de médicaments, les produits de formule générale (I) telle que définie à l'une des revendications 2 à 5, ou leurs sels pharmaceutiquement acceptables.

**10.** Les compositions pharmaceutiques comprenant, comme principe actif au moins un médicament défini à l'une quelconque des revendications 8 ou 9.

**Revendications pour les Etats contractants suivants : ES, GR**

**1.** Procédé de préparation des produits de formule générale (I) :

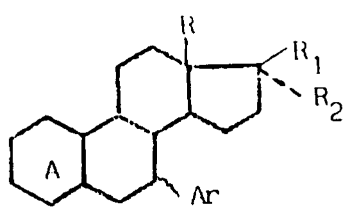

(I)

dans laquelle le cycle A présente l'une des structures suivantes :

a/. soit A représente le groupement :

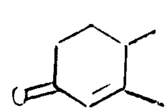

b/. soit A représente le groupement :

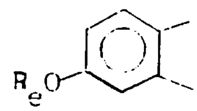

dans lequel Re représente un atome d'hydrogène, un radical alkyle ayant de 1 à 6 atomes de carbone éventuellement substitué ou un radical acyle,
R représente un radical méthyle ou éthyle,
$R_1$ représente un radical hydroxyle éventuellement protégé ou acylé par un radical acyle choisi parmi les radicaux acétyle, chloroacétyle ou trifluoroacétyle, ou $R_1$ représente un radical alcoxyle,
$R_2$ représente un atome d'hydrogène, un radical alkyle, alkényle ou alkynyle ayant au plus 8 atomes de carbone ou un radical aryle ou aralkyle ayant au plus 15 atomes de carbone, chacun de ces radicaux alkyle, alkényle, alkynyle, aryle ou aralkyle étant éventuellement substitué, par un ou plusieurs substituants identiques ou différents choisis parmi les radicaux :

- halogène,
- alkoxy,
- alkylthio,
- amino, alkylamino, dialkylamino, chacun des radicaux dialkylamino étant éventuellement sous forme oxydée,
- aminoalkyl,
- dialkylaminoalkyle,
- dialkylaminoalkyloxy,
- hydroxyle, acétoxy, un radical de formule

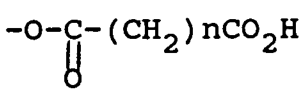

dans lequel n = 2 à 5,
- acyle,
- carboxy libre ou estérifié,
- cyano,
- trifluorométhyle,
- aryle ou aralkyle,

les radicaux aryle et aralkyle, que peut représenter $R_2$ pouvant être de plus substitués par un radical alkyle,
soit $R_1$ et $R_2$ forment ensemble un radical choisi parmi les radicaux :

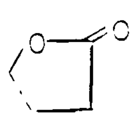 ; 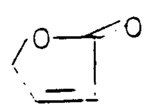 ; 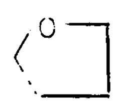 ; 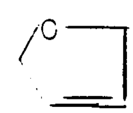

Ar représente un radical aryle ayant 5 ou 6 chaînons carbocyclique ou hétérocyclique, choisi parmi les radicaux thiényle, furyle, thiazolyle, pyrrolyle, oxazolyle, imidazolyle, pyrazolyle, triazolyle (1,2,3 ou 1,2,4) tétrazolyle, isothiazolyle, isoxazolyle, thiadiazolyle, phényle, pyridinyle, pyridazinyle, pyrimidinyle, pyrazinyle, pipéridinyle,
ces radicaux étant éventuellement substitués par un ou plusieurs substituants identiques ou différents choisis parmi les radicaux :

- halogène,
- alkyle,
- alkoxy,
- alkylthio,

- amino, alkylamino, dialkylamino, chacun des radicaux dialkylamino étant éventuellement sous forme oxydée,
- aminoalkyl,
- dialkylaminoalkyle,
- dialkylaminoalkyloxy,
- hydroxyle, acétoxy, un radical de formule

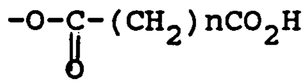

dans lequel n = 2 à 5,
- acyle,
- carboxy libre ou estérifié,
- cyano,
- trifluorométhyle,
- aryle ou aralkyle,

ainsi que les sels d'addition des produits de formule (I) avec les acides et les bases, caractérisé en ce l'on soumet un produit de formule (II) :

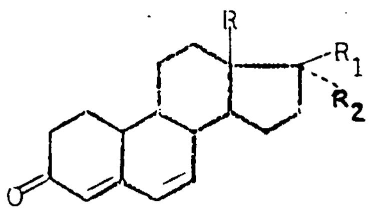

(II)

dans laquelle R, $R_1$ et $R_2$ ont la signification indiquée précédemment d'abord à l'action, éventuellement en présence d'un acide de Lewis,
- soit d'un produit de formule ($III_a$) :

Ar-Mg-Hal ($III_a$)

dans laquelle Ar a la signification indiquée précédemment et Hal représente un atome d'halogène en présence d'un sel cuivreux :
- soit d'un produit de formule ($III_b$) :

$Ar_2CuLi$ ($III_b$)

- soit d'un produit de formule ($III_c$) :

$Ar_2CuCNLi_2$ ($III_c$)

pour obtenir un produit de formule ($I_A$) :

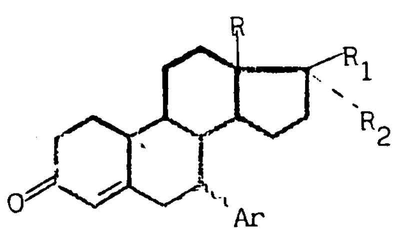

($I_A$)

éventuellement sous forme d'un mélange d'isomères 7 alpha et 7 béta que l'on sépare, si désiré et

produit de formule $I_A$ correspondant à un produit de formule (I) dans laquelle le cycle A représente le groupement :

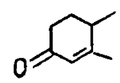

que l'on traite, si désiré par un agent d'aromatisation pour obtenir un produit de formule

$(I_{B_1})$ :

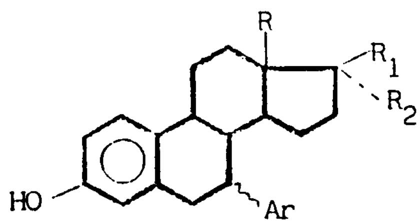

$(I_{B_1})$

dans laquelle Ar, R, $R_1$ et $R_2$ ont la signification précédemment et correspondant à un produit de formule I dans laquelle le cycle A représente le groupement :

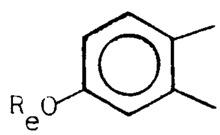

Re représentant un atome d'hydrogène et produit de formule

$(I_{B_1})$

que l'on traite si désiré par un réactif d'alkylation pour obtenir un produit de formule

$(I_{B_2})$ :

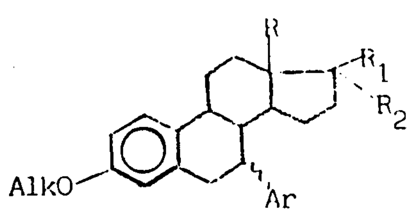

$(I_{B_2})$

dans laquelle Alk représente un radical alkyle ayant de 1 à 6 atome de carbone éventuellement substitué et correspondant à un produit de formule (I) dans lequel le cycle A représente le groupement :

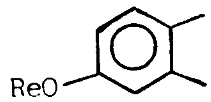

Re représentant un radical alkyle éventuellement substitué, produits de formules ($I_A$),

($I_{B_1}$) ou ($I_{B_2}$)

que, selon la valeur des substituants $R_2$ et Ar l'on soumet si désiré à l'action d'une base ou d'un acide pour obtenir les sels correspondants.

**2.** Procédé selon la revendication 1, caractérisé en ce que l'on soumet un produit de formule ($I_C$) :

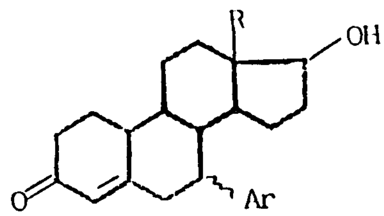

($I_C$)

dans laquelle R et Ar ont la signification indiquée précédemment, correspondant à un produit de formule ($I_A$) dans laquelle $R_1$ représente un radical hydroxyle et $R_2$ représente un atome d'hydrogène à l'action d'un réactif de blocage de la fonction 3-céto par exemple-un éther d'énol ou un cétal- pour obtenir un produit de formule (IV) :

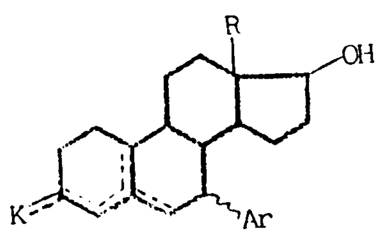

(IV)

dans laquelle K et les traits pointillés représentent une fonction cétonique protégée, de préférence un produit de formules ($IV_A$) ou ($IV_B$) :

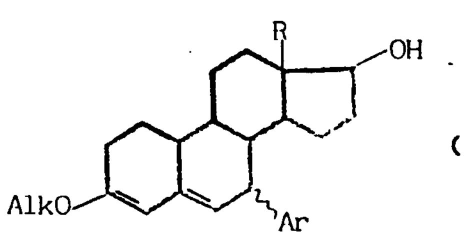

($IV_A$)

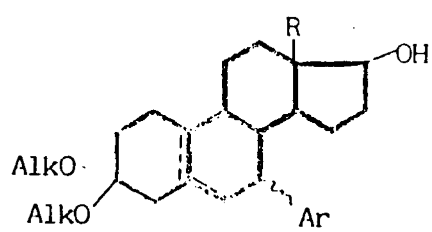

($VI_B$)

dans laquelle Alk représente de préférence un radical akyle et les traits pointillés représentent une double liaison en 5(6) ou 5(10), produit de formule (IV) que l'on traite par un agent d'oxydation pour obtenir un produit de formule (V) :

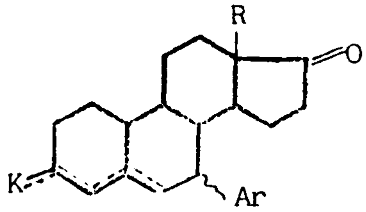

(V)

produit sur lequel on fait agir soit un dérivé organo métallique dérivé d'un radical $R_2$ représentant un radical alkyle, alkényle, alkynyle ayant au plus 8 atomes de carbone ou un radical aryle ou aralkyle ayant au plus 15 atomes de carbone, chacun de ces radicaux alkyle, alkényle, alkynyle, aryle ou aralkyle étant éventuellement substitué, soit d'abord un dérivé organométallique dérivé du réactif de formule $-C{\equiv}C-CH_2OH$ dans lequel la fonction hydroxyle est éventuellement protégée, puis dans un ordre quelconque si désiré à un réactif de déprotection et à un réactif de réduction totale ou partielle, puis à un réactif de cyclisation ou d'oxydation, soit d'abord à un réactif de formule

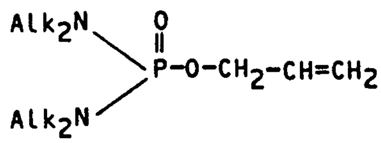

en présence d'une base forte pour obtenir respectivement les produits de formule ($VI_A$) :

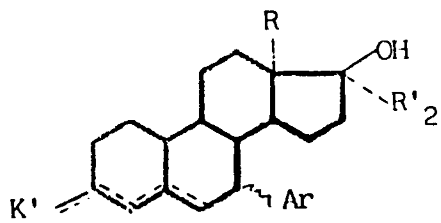

($VI_A$)

dans laquelle $R'_2$ a les valeurs de $R_2$ à l'exception de la valeur hydrogène et K′ et les traits pointillés représentent une fonction 3-céto delta 4 éventuellement protégée et les produits de formule ($VI_B$) :

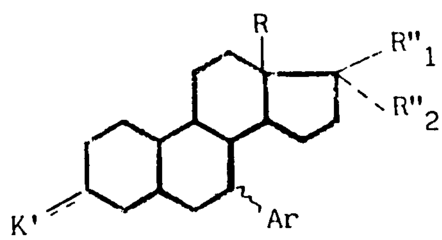

($VI_B$)

dans laquelle $R''_1$ et $R''_2$ représentent ensemble un radical :

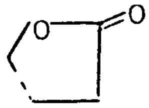 ; 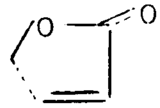 , 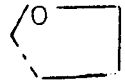 ou 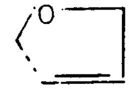

produits de formules ($VI_A$) et ($VI_B$) que l'on soumet le cas échéant à un réactif de déprotection lorsque K′représente un groupement protecteur de la fonction 3-céto delta-4 pour obtenir les produits de formule ($I_D$) :

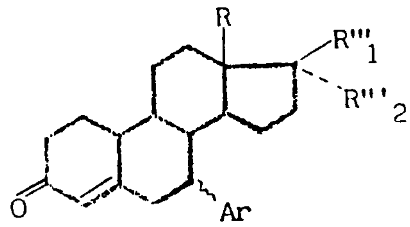

$(I_D)$

dans laquelle $R'''_1$ et $R'''_2$ ont les valeurs indiquées ci-dessus pour $R_1$ et $R_2$ à l'exception de la valeur $R_2$ = hydrogène, produits de formule ($I_D$) que l'on peut le cas échéant, lorsque $R'''_1$ représente un radical hydroxyle, soumettre à l'action d'un réactif de protection, d'acylation ou d'alkylation pour obtenir un produit ($I'_D$) correspondant dans lequel la fonction 17béta-OH est protégée, acylée ou alkylée, produit de formules ($I_D$ et ($I'_D$) correspondant à des produits de formule ($I_A$), que l'on peut le cas échéant traiter comme indiqué à la revendication 1.

**3.** Procédé selon la revendication 1 ou 2, caractérisé en ce que l'on prépare des produits de formule (I), dans laquelle le cycle A représente le groupement :

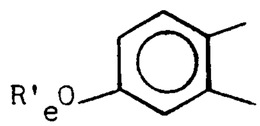

dans lequel $R'_e$ représente un atome d'hydrogène ou un radical alkyle ayant de 1 à 4 atomes de carbone.

**4.** Procédé selon l'une quelconque des revendications 1 à 3, caractérisé en ce que l'on utilise au départ un produit de formule ($III_a$), ($III_b$) ou ($III_c$) ou un produit de formule ($I_C$) dans laquelle, Ar représente :
a/. soit un radical phényle éventuellement substitué par un ou plusieurs substituants choisis parmi les radicaux halogène, alkyle, alkoxy ou alkylthio ayant de 1 à 4 atomes de carbone, amino, alkylamino, dialkylamino, dialkylamino alkoxyle, hydroxyle, acyle, carboxy libre estérifié ou salifié, cyano, trifluorométhyle, phényle ou benzyle eux-mêmes éventuellement substitués par un ou plusieurs radicaux alkyles ayant de 1 à 4 atomes de carbone,
b/. soit un radicalhétérocyclique choisi parmi les radicaux thiényle, furyle, thiazolyle, isothiazolyle, oxazolyle, isoxazolyle, thiadiazolyle, pyridinyle, piperidinyle.

**5.** Procédé selon la revendication 1 ou 3, caractérisé en ce que l'on utilise au départ un produit de formule (II) dans laquelle $R_1$ représente un radical hydroxyle éventuellement protégé ou acylé, $R_2$ représente un radical alkyle, alkényle ou alkynyle, chacun de ces radicaux étant éventuellement substitué par un radical choisi parmi les halogènes, les radicaux hydroxyle, carboxy éventuellement estérifié ou salifié ou cyano.

**6.** Procédé selon l'une quelconque des revendications 1 à 5, caractérisé en de que l'on prépare :
- Le 7alpha-[4-[2-(diméthylamino) éthoxy] phényl] estra-1,3,5(10)-trièn-3,17béta-diol.
- La 7alpha-[4-[2-(diméthylamino) éthoxy] phényl] 17béta-hydroxy estr-4-èn-3-one.
- Le 7béta-[4-[2-(diméthylamino) éthoxy] phényl] estra-1,3,5(10)-trièn-3,17béta-diol.
- La 7béta-[4-(diméthylamino) éthoxy] phényl] 17béta-hydroxy estr-4-èn-3-one.
- Le 7alpha -[4-[2-(diméthylamino) phényl] estra-1,3,5(10)-trièn-3,17 béta-diol.
- Le 7alpha -(4-méthoxyphényl) estra-1,3,5(10)-trièn-3,17béta-diol.
- La 17béta-hydroxy 7alpha-[4-(méthylthio) phényl] estr-4-èn-3-one.
- Le 7alpha-[4-(méthylthio) phényl] estra-1,3,5(10)-trién-3,17béta-diol.

**7.** Procédé de préparation de compositions pharmaceutiques, caractérisé en ce que l'on met à titre de principe actif l'un au moins des composés de formule (I), telle que définie à la revendication 1 ou l'un au moins de leurs sels pharmaceutiquement acceptables, sous une forme destinée à cet usage.

**8.** Procédé de préparation de compositions pharmaceutiques, caractérisé en ce que l'on met à titre de principe actif l'un au moins des composés de formule (I), telle que définie à la revendication 11 ou l'un au moins de leurs sels pharmaceutiquement acceptables, sous une forme destinée à cet usage.

**Claims**

**Claims for the following Contracting States : AT, BE, CH, DE, FR, GB, IT, LI, LU, NL, SE**

**1.** The products of general formula (I):

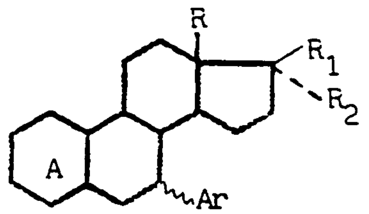

(I)

in which the ring A has one of the following structures:

a. either A represents the group:

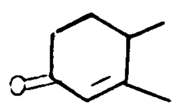

b. or A represents the group:

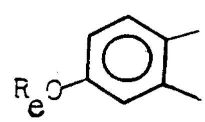

in which Re represents a hydrogen atom, an optionally substituted alkyl radical having 1 to 6 carbon atoms or an acyl radical,

R represents a methyl or ethyl radical,

$R_1$ represents a hydroxyl radical optionally protected or acylated by an acyl radical chosen from the acetyl, chloroacetyl or trifluoracetyl radicals, or $R_1$ represents an alkoxyl radical, $R_2$ represents a hydrogen atom, an alkyl, alkenyl or alkynyl radical having at most 8 carbon atoms or an aryl or aralkyl radical having at most 15 carbon atoms, each of these alkyl, alkenyl, alkynyl, aryl or aralkyl radicals being optionally substituted by one or more identical or different substituents chosen from the following radicals:

- halogen,
- alkoxy,
- alkylthio,
- amino, alkylamino, dialkylamino, each of these dialkylamino radicals being optionally in an oxidized form,
- aminoalkyl,
- dialkylaminoalkyl,
- dialkylaminoalkyloxy,
- hydroxyl, acetoxy, a radical of formula

$$-O-\underset{\underset{O}{\|}}{C}-(CH_2)nCO_2H$$

in which n = 2 to 5,

- acyl,
- free or esterified carboxy,
- cyano,
- trifluoromethyl,
- aryl or aralkyl,

the aryl and aralkyl radicals which can be represented by $R_2$ can be moreover substituted by an alkyl radical,
or $R_1$ and $R_2$ form together a radical chosen from the following radicals:

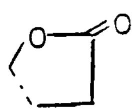 ; 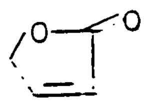 ; 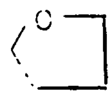 ; 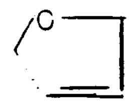

Ar represents an aryl radical having 5 or 6 links, carbocyclic or heterocyclic, chosen from the following radicals: thienyl, furyl, thiazolyl, pyrrolyl, oxazolyl, imidazolyl, pyrazolyl, triazolyl (1,2,3 or 1,2,4), tetrazolyl, isothiazolyl, isoxazolyl, thiadiazolyl, phenyl, pyridinyl, pyridazinyl, pyrimidinyl, pyrazinyl, piperidinyl, these radicals being optionally substituted by one or more identical or different substituents chosen from the following radicals:

- halogen,
- alkyl,
- alkoxy,
- alkylthio,
- amino, alkylamino, dialkylamino, each of these dialkylamino radicals being optionally in an oxidized form,
- aminoalkyl,
- dialkylaminoalkyl,
- dialkylaminoalkyloxy,
- hydroxyl, acetoxy, a radical of formula

$$-O-\underset{\underset{O}{\|}}{C}-(CH_2)nCO_2H$$

in which n = 2 to 5,

- acyl,
- free or esterified carboxy,
- cyano,
- trifluoromethyl,
- aryl or aralkyl,

as well as the addition salts of the products of formula (I) with acids and bases.

**2.** The products of formula (I) as defined in claim 1, in which the ring A represents the group:

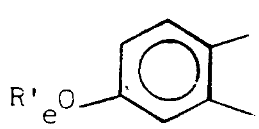

in which $R'_e$ represents a hydrogen atom or an alkyl radical having 1 to 4 carbon atoms.

**3.** The products of formula (I) as defined in one of claims 1 or 2, in which Ar represents:
a) either a phenyl radical optionally substituted by one or more substituents chosen from the following radicals: halogen, alkyl, alkoxy or alkylthio having 1 to 4 carbon atoms, amino, alkylamino, dialkylamino, dialkylamino alkoxyl, hydroxyl, acyl, free, esterified or salified carboxy, cyano, trifluoromethyl, phenyl or benzyl, themselves optionally substituted by one or more alkyl radicals having 1 to 4 carbon atoms,
b) or a heterocyclic radical chosen from the following radicals: thienyl, furyl, thiazolyl, isothiazolyl, oxazolyl, isoxazolyl, thiadiazolyl, pyridinyl, piperidinyl.

**4.** The products of formula (I) as defined in one of claims 1 to 3, in which $R_1$ represents an optionally protected or acylated hydroxyl radical, $R_2$ represents an alkyl, alkenyl or alkynyl radical, each of these radicals being optionally substituted by a radical chosen from halogens, hydroxyl, optionally esterified or salified carboxy or cyano radicals.

**5.** The products of general formula (I), as defined in one of claims 1 to 4, of which the names follow:
- 7alpha-[4-[2-(dimethylamino)-ethoxy]-phenyl]-estra-1,3,5(10)-trien-3,17beta-diol.
- 7alpha-[4-[2-(dimethylamino)-ethoxy]-phenyl]-17beta-hydroxy-estr-4-en-3-one.
- 7beta-[4-[2-(dimethylamino)-ethoxy]-phenyl]-estra-1,3,5(10)-trien-3,17beta-diol.
- 7beta-[4-(dimethylamino)-ethoxy]-phenyl]-17beta-hydroxy-estr-4-en-3-one.
- 7 alpha-[4-[2-(dimethylamino)-phenyl]-estra-1,3,5(10)-trien-3,17-beta-diol.
- 7alpha-(4-methoxyphenyl)-estra-1,3,5(10)-trien-3,17beta-diol.
- 17beta-hydroxy-7alpha-[4-(methylthio)-phenyl]-estr-4-en-3-one.
- 7alpha-[4-(methylthio)-phenyl]-estra-1,3,5(10)-trien-3,17beta-diol.

**6.** Preparation process for the products of general formula (I) as defined in claim 1, characterized in that a product of formula (II):

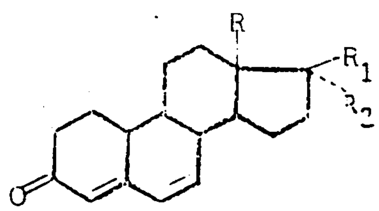

(II)

in which R, $R_1$ and $R_2$ have the meaning indicated in claim 1 is first subjected to the action, optionally in the presence of a Lewis acid,
- <u>either</u> of a product of formula ($III_a$):

  Ar-Mg-Hal ($III_a$)

  in which Ar has the meaning indicated in claim 1 and Hal represents a halogen atom, in the presence of a cuprous salt:
- <u>or</u> of a product of formula ($III_b$):

  $Ar_2CuLi$ ($III_b$)
- <u>or</u> of a product of formula ($III_c$)

  $Ar_2CuCNLi_2$ ($III_c$)

in order to obtain a product of formula ($I_A$):

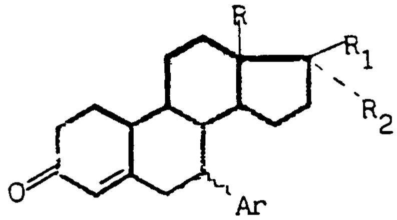

$(I_A)$

optionally in the form of a mixture of 7alpha and 7beta isomers which are separated, if desired, and the product of formula ($I_A$) corresponding to a product of formula (I) in which the ring A represents the group:

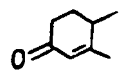

which is treated, if desired, by an aromatisation agent in order to obtain a product of formula

$(I_{B_1})$ :

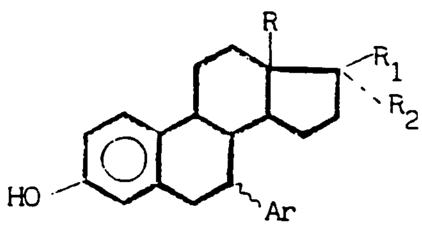

$(I_{B_1})$

in which Ar, R, $R_1$ and $R_2$ have the meaning indicated in claim 1 and corresponding to a product of formula I in which the ring A represents the group:

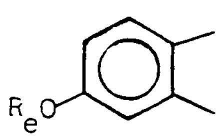

Re representing a hydrogen atom and which product of formula

$(I_{B_1})$

is treated, if desired, with an alkylation reagent in order to obtain a product of formula

$(I_{B_2})$ :

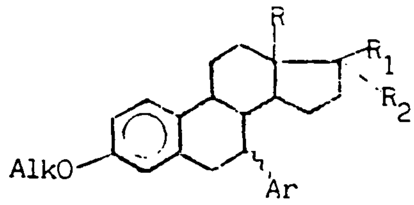

$(I_{B_2})$

in which Alk represents an alkyl radical having 1 to 6 carbon atoms optionally substituted and corresponding to a product of formula (I) in which the ring A represents the group:

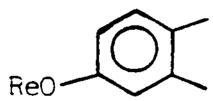

Re representing an optionally substituted alkyl radical, which products of formulae $(I_A)$,

$(I_{B_1})$ or $(I_{B_2})$,

are, according to the value of substituents $R_2$ and Ar, subjected, if desired, to the action of a base or an acid in order to obtain the corresponding salts.

**7.** Process according to claim 6, characterized in that a product of formula $(I_C)$:

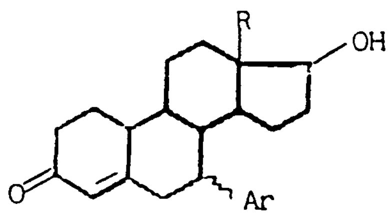

$(I_C)$

in which R and Ar have the meaning indicated in claim 1, corresponding to a product of formula $(I_A)$ in which $R_1$ represents a hydroxyl radical and $R_2$ represents a hydrogen atom, is subjected to the action of a blocking reagent of the 3-keto function, for example an enol ether or a ketal, in order to obtain a product of formula (IV):

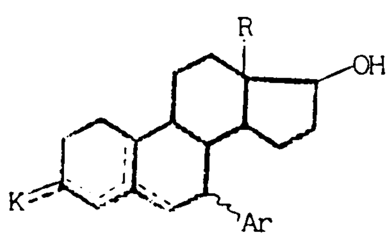

(IV)

in which K and the dotted lines represent a protected ketone function, preferably a product of formula $(IV_A)$ or $(IV_B)$:

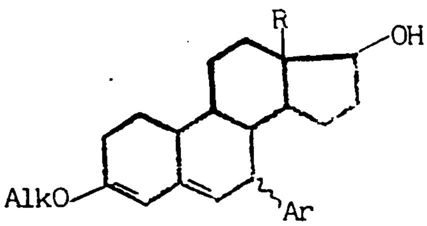 (IV$_A$) 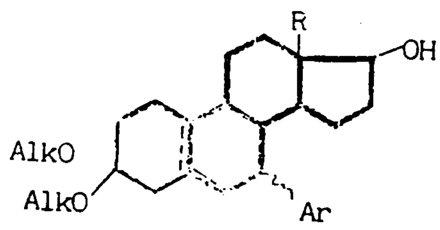 (IV$_B$)

in which Alk preferably represents an alkyl radical and the dotted lines represent a double bond in position 5(6) or 5(10), which product of formula (IV) is treated with an oxidizing agent in order to obtain a product of formula (V):

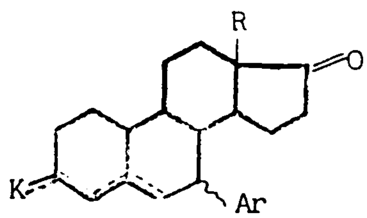

(V)

on which product the following are reacted: either an organometallic derivative derived from an $R_2$ radical representing an alkyl, alkenyl, alkynyl radical having at most 8 carbon atoms or an aryl or aralkyl radical having at most 15 carbon atoms, each of these alkyl, alkenyl, alkynyl aryl or aralkyl radicals being optionally substituted, or firstly an organometallic derivative derived from a reagent of formula $-C \equiv C-CH_2OH$ in which the hydroxyl function is optionally protected, then if desired, in any order, to a deprotection reagent and to a total or partial reducing reagent, then to a cyclization or oxidizing reagent, or firstly to a reagent of formula:

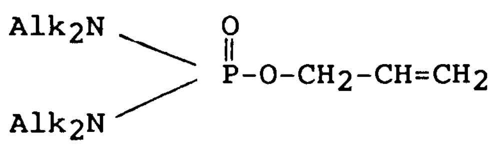

in the presence of a strong base in order to obtain respectively the products of formula (VI$_A$):

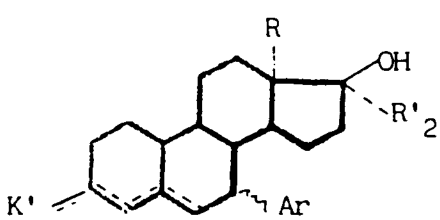

(VI$_A$)

in which $R'_2$ has the values of $R_2$ with the exception of the hydrogen value and K' and the dotted lines represent an optionally protected 3-keto-delta 4 function and the products of formula (VI$_B$):

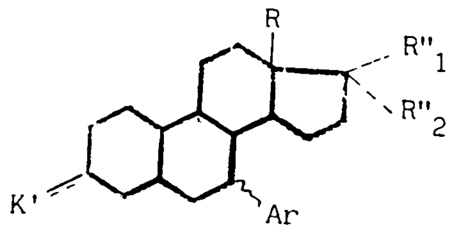

(VI$_B$)

in which R"$_1$ and R"$_2$ together represent a radical:

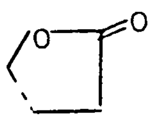 , 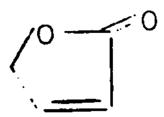 , 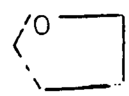 or 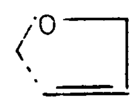

which products of formulae (VI$_A$) and (VI$_B$) are subjected, if appropriate, to a deprotection reagent when K' represents a protective group of the 3-keto-delta-4 function in order to obtain the products of formula (I$_D$):

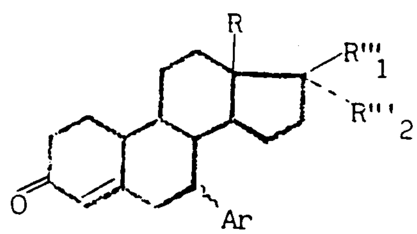

(I$_D$)

in which R'"$_1$ and R'"$_2$ have the values indicated above for R$_1$ and R$_2$ with the exception of the value R$_2$ = hydrogen, which products of formula (I$_D$) can, if appropriate, when R'"$_1$ represents a hydroxyl radical, be subjected to the action of a protection, acylation or alkylation reagent in order to obtain a corresponding product (I'$_D$) in which the 17beta-OH function is protected, acylated or alkylated, which product of formulae (I$_D$) and (I'$_D$) corresponding to the products of formula (I$_A$), can, if appropriate, can be treated as indicated in claim 1.

**8.** As medicaments, the products of general formula (I) as defined in claim 1, or their pharmaceutically acceptable salts.

**9.** As medicaments, the products of general formula (I) as defined in one of claims 2 to 5, or their pharmaceutically acceptable salts.

**10.** The pharmaceutical compositions containing, as active ingredient, at least one medicament defined in any one of claims 8 or 9.

**Claims for the following Contracting States : ES, GR**

**1.** Preparation process for the products of general formula (I):

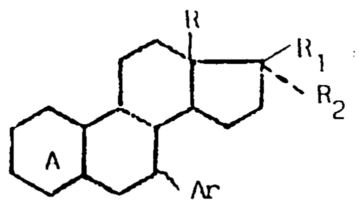

(I)

in which the ring A has one of the following structures:

a. either A represents the group:

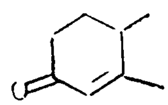

b. or A represents the group:

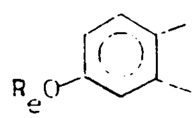

in which Re represents a hydrogen atom, an optionally substituted alkyl radical having 1 to 6 carbon atoms or an acyl radical,

R represents a methyl or ethyl radical,

$R_1$ represents a hydroxyl radical optionally protected or acylated by an acyl radical chosen from the acetyl, chloroacetyl or trifluoracetyl radicals, or $R_1$ represents an alkoxyl radical, $R_2$ represents a hydrogen atom, an alkyl, alkenyl or alkynyl radical having at most 8 carbon atoms or an aryl or aralkyl radical having at most 15 carbon atoms, each of these alkyl, alkenyl, alkynyl, aryl or aralkyl radicals being optionally substituted by one or more identical or different substituents chosen from the following radicals:

- halogen,
- alkoxy,
- alkylthio,
- amino, alkylamino, dialkylamino, each of these dialkylamino radicals being optionally in an oxidized form,
- aminoalkyl,
- dialkylaminoalkyl,
- dialkylaminoalkyloxy,
- hydroxyl, acetoxy, a radical of formula

$$-O-C(=O)-(CH_2)nCO_2H$$

in which n = 2 to 5,

- acyl,
- free or esterified carboxy,
- cyano,
- trifluoromethyl,
- aryl or aralkyl,

the aryl and aralkyl radicals which can be represented by $R_2$ can be moreover substituted by an alkyl radical,

or $R_1$ and $R_2$ form together a radical chosen from the following radicals:

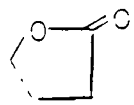 , 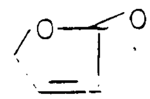 ; 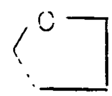 ; 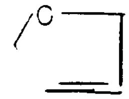

Ar represents an aryl radical having 5 or 6 links, carbocyclic or heterocyclic, chosen from the following radicals: thienyl, furyl, thiazolyl, pyrrolyl, oxazolyl, imidazolyl, pyrazolyl, triazolyl (1,2,3 or 1,2,4), tetrazolyl, isothiazolyl, isoxazolyl, thiadiazolyl, phenyl, pyridinyl, pyridazinyl, pyrimidinyl, pyrazinyl, piperidinyl, these radicals being optionally substituted by one or more identical or different substituents chosen from the following radicals:

- halogen,
- alkyl,
- alkoxy,
- alkylthio,
- amino, alkylamino, dialkylamino, each of these dialkylamino radicals being optionally in an oxidized form,
- aminoalkyl,
- dialkylaminoalkyl,
- dialkylaminoalkyloxy,
- hydroxyl, acetoxy, a radical of formula

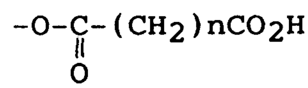

  in which n = 2 to 5,
- acyl,
- free or esterified carboxy,
- cyano,
- trifluoromethyl,
- aryl or aralkyl,

as well as the addition salts of the products of formula (I) with acids and bases, characterized in that a product of formula (II):

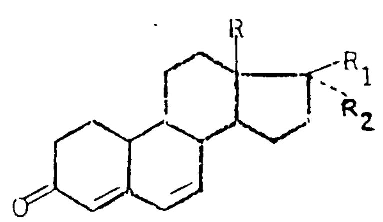

(II)

in which R, $R_1$ and $R_2$ have the meaning indicated in claim 1 is first subjected to the action, optionally in the presence of a Lewis acid,

- either of a product of formula ($III_a$):

  Ar-Mg-Hal (IIIa)

  in which Ar has the meaning indicated in claim 1 and Hal represents a halogen atom, in the presence of a cuprous salt:
- or of a product of formula ($III_b$):

  $Ar_2CuLi$ ($III_b$)

- or of a product of formula ($III_c$):

$Ar_2CuCNLi_2$ ($III_c$)

in order to obtain a product of formula ($I_A$):

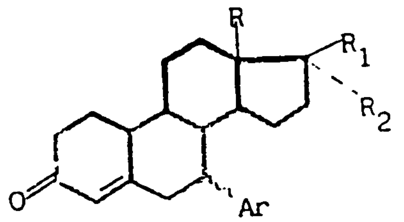

($I_A$)

optionally in the form of a mixture of 7alpha and 7beta isomers which are separated, if desired, and the product of formula ($I_A$) corresponding to a product of formula (I) in which the ring A represents the group:

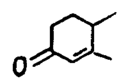

which is treated, if desired, by an aromatisation agent in order to obtain a product of formula

($I_{B_1}$) :

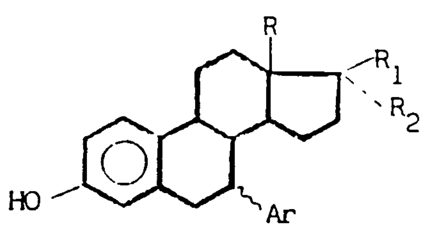

($I_{B_1}$)

in which Ar, R, $R_1$ and $R_2$ have the meaning indicated previously and corresponding to a product of formula I in which the ring A represents the group:

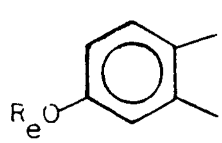

Re representing a hydrogen atom and which product of formula

($I_{B_1}$)

is treated, if desired, with an alkylation reagent in order to obtain a product of formula

$(I_{B_2})$ :

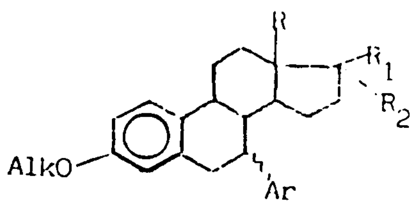

$(I_{B_2})$,

in which Alk represents an alkyl radical having 1 to 6 carbon atoms optionally substituted and corresponding to a product of formula (I) in which the ring A represents the group:

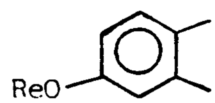

Re representing an optionally substituted alkyl radical, which products of formulae $(I_A)$,

$(I_{B_1})$ or $(I_{B_2})$,

are, according to the value of substituents $R_2$ and Ar, subjected, if desired, to the action of a base or an acid in order to obtain the corresponding salts.

**2.** Process according to claim 1, characterized in that a product of formula $(I_C)$:

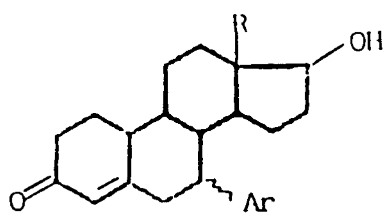

$(I_C)$

in which R and Ar have the meaning indicated previously, corresponding to a product of formula $(I_A)$ in which $R_1$ represents a hydroxyl radical and $R_2$ represents a hydrogen atom, is subjected to the action of a blocking reagent of the 3-keto function for example an enol ether or a ketal, in order to obtain a product of formula (IV):

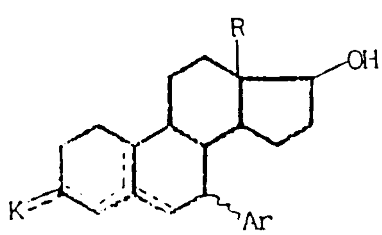

(IV)

in which K and the dotted lines represent a protected ketone function, preferably a product of formula $(IV_A)$ or $(IV_B)$:

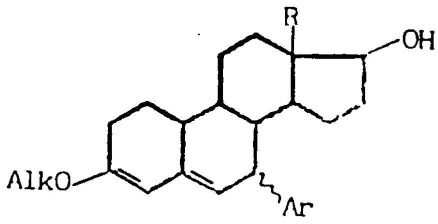

(IV$_A$)

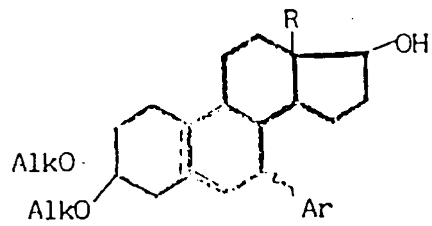

(IV$_B$)

in which Alk preferably represents an alkyl radical and the dotted lines represent a double bond in position 5(6) or 5(10), which product of formula (IV) is treated with an oxidizing agent in order to obtain a product of formula (V):

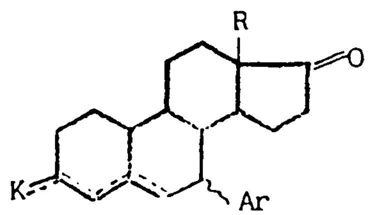

(V)

on which product the following are reacted: either an organometallic derivative derived from an $R_2$ radical representing an alkyl, alkenyl, alkynyl radical having at most 8 carbon atoms or an aryl or aralkyl radical having at most 15 carbon atoms, each of these alkyl, alkenyl, alkynyl aryl or aralkyl radicals being optionally substituted, or firstly an organometallic derivative derived from a reagent of formula $-C \equiv C-CH_2OH$ in which the hydroxyl function is optionally protected, then if desired, in any order, to a deprotection reagent and to a total or partial reducing reagent, then to a cyclization or oxidizing reagent, or firstly to a reagent of formula:

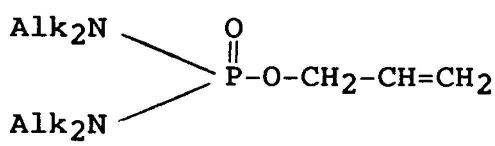

in the presence of a strong base in order to obtain respectively the products of formula (VI$_A$):

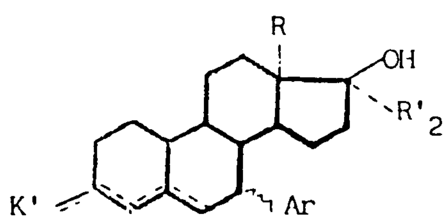

(VI$_A$)

in which $R'_2$ has the values of $R_2$ with the exception of the hydrogen value and K' and the dotted lines represent an optionally protected 3-keto-delta 4 function and the products of formula (VI$_B$):

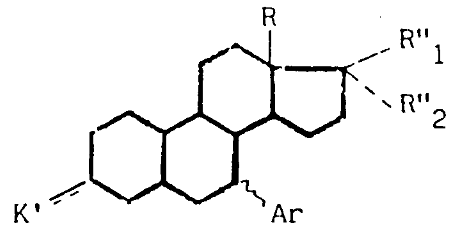

(VI$_B$)

in which R"$_1$ and R"$_2$ together represent a radical:

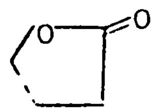 ; 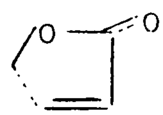 ; 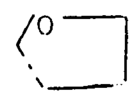 or 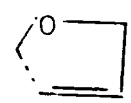

which products of formulae (VI$_A$) and (VI$_B$) are subjected, if appropriate, to a deprotection reagent when K' represents a protector group of the 3-keto-delta-4 function, in order to obtain the products of formula (I$_D$):

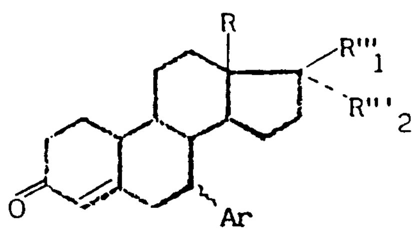

(I$_D$)

in which R'"$_1$ and R'"$_2$ have the values indicated above for R$_1$ and R$_2$ with the exception of the value R$_2$ = hydrogen, which products of formula (I$_D$) can, if appropriate, when R'"$_1$ represents a hydroxyl radical, be subjected to the action of a protection, acylation or alkylation reagent in order to obtain the corresponding product (I'$_D$) in which the 17beta-OH function is protected, acylated or alkylated, which product of formulae (I$_D$) and (I'$_D$) corresponding to the products of formula (I$_A$), can, if appropriate, be treated as indicated in claim 1.

**3.** Process according to claim 1 or 2, characterized in that products of formula (I) are prepared, in which the ring A represents the group:

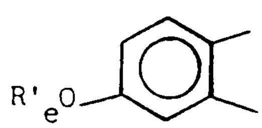

in which R'$_e$ represents a hydrogen atom or an alkyl radical having 1 to 4 carbon atoms.

**4.** Process according to any one of claims 1 to 3, characterized in that a product of formula (III$_a$), (III$_b$) or (III$_c$) or a product of formula (I$_C$) is used at the start in which Ar represents:

a) either a phenyl radical optionally substituted by one or more substituents chosen from the following radicals: halogen, alkyl, alkoxy or alkylthio having 1 to 4 carbon atoms, amino, alkylamino, dialkylamino, dialkylamino alkoxyl, hydroxyl, acyl, free, esterified or salified carboxy, cyano, trifluoromethyl, phenyl or benzyl, themselves optionally substituted by one or more alkyl radicals having 1 to 4 carbon atoms,

b) or a heterocyclic radical chosen from the following radicals: thienyl, furyl, thiazolyl, isothiazolyl, oxazolyl, isoxazolyl, thiadiazolyl, pyridinyl, piperidinyl.

**5.** Process according to claim 1 or 3, characterized in that a product of formula (II) is used at the start in which $R_1$ represents an optionally protected or acylated hydroxyl radical, $R_2$ represents an alkyl, alkenyl or alkynyl radical, each of these radicals being optionally substituted by a radical chosen from halogens, hydroxyl, optionally esterified or salified carboxy or cyano radicals.

**6.** Process according to any one of claims 1 to 5, characterized in that the following are prepared:
- 7alpha-[4-[2-(dimethylamino)-ethoxy]-phenyl]-estra-1,3,5(10)-trien-3,17beta-diol.
- 7alpha-[4-[2-(dimethylamino)-ethoxy]-phenyl]-17beta-hydroxy-estr-4-en-3-one.
- 7beta-[4-[2-(dimethylamino)-ethoxy]-phenyl]-estra-1,3,5(10)-trien-3,17beta-diol.
- 7beta-[4-(dimethylamino)-ethoxy]-phenyl]-17beta-hydroxy-estr-4-en-3-one.
- 7 alpha-[4-[2-(dimethylamino)-phenyl]-estra-1,3,5(10)0 trien-3,17-beta-diol.
- 7alpha-(4-methoxyphenyl)-estra-1,3,5(10)-trien-3,17beta-diol.
- 17beta-hydroxy-7alpha-[4-(methylthio)-phenyl]-estr-4-en-3-one.
- 7alpha-[4-(methylthio)-phenyl]-estra-1,3,5(10)-trien-3,17beta-diol.

**7.** Preparation process for pharmaceutical compositions, characterized in that at least one of the compounds of formula (I) as defined in claim 1 or at least one of their pharmaceutically acceptable salts is used as active ingredient, in a form intended for this use.

**8.** Preparation process for pharmaceutical compositions, characterized in that at least one of the compounds of formula (I) as defined in claim 11 or at least one of their pharmaceutically acceptable salts is used as active ingredient, in a form intended for this use.

**Patentansprüche**
**Patentansprüche für folgende Vertragsstaaten : AT, BE, CH, DE, FR, GB, IT, LI, LU, NL, SE**

**1.** Produkte der allgemeinen Formel (I)

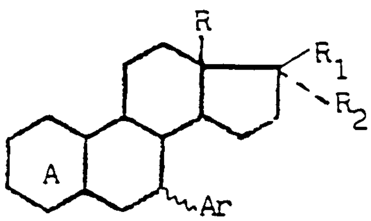

(I)

worin der Ring A eine der folgenden Strukturen besitzt:
(a) entweder bedeutet A die Gruppe

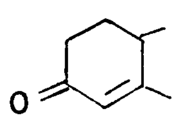

(b) oder A bedeutet die Gruppe

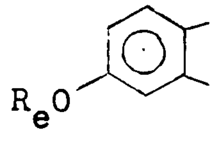

worin Re ein Wasserstoffatom, eine Alkylgruppe mit 1 bis 6 Kohlenstoffatomen, die gegebenenfalls substituiert ist, oder einen Acylrest bedeutet,
R für einen Methyl- oder Ethylrest steht,
$R_1$ eine Hydroxylgruppe bedeutet, die gegebenenfalls geschützt oder mit einem Acylrest, ausgewählt unter den Acetyl-, Chloracetyl- oder Trifluoracetylresten, acyliert ist, oder $R_1$ einen Alkoxyrest bedeutet,

$R_2$ ein Wasserstoffatom, einen Alkyl-, Alkenyl- oder Alkinylrest mit höchstens 8 Kohlenstoffatomen oder einen Aryl- oder Aralkylrest mit höchstens 15 Kohlenstoffatomen wiedergibt, wobei ein jeder dieser Alkyl-, Alkenyl-, Alkinyl-, Aryl- oder Aralkylreste gegebenenfalls durch einen oder mehrere gleiche oder verschiedene Substituenten substituiert ist, ausgewählt unter den Resten:

- Halogen,
- Alkoxy,
- Alkylthio,
- Amino, Alkylamino, Dialkylamino, wobei ein jeder der Dialkylaminoreste gegebenenfalls in oxidierter Form vorliegt,
- Aminoalkyl,
- Dialkylaminoalkyl,
- Dialkylaminoalkoxy,
- Hydroxyl, Acetoxy, einem Rest der Formel

$$-O-\underset{\underset{O}{\parallel}}{C}-(CH_2)_nCO_2H,$$

  worin n = 2 bis 5,
- Acyl, - freies oder verestertes Carboxy,
- Cyano,
- Trifluormethyl,
- Aryl oder Aralkyl,

wobei die Aryl- und Aralkylreste, die $R_2$ wiedergeben kann, außerdem durch einen Alkylrest substituiert sein können, oder $R_1$ und $R_2$ gemeinsam einen Rest bilden, ausgewählt unter den Resten

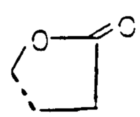 . 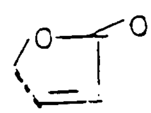 ; 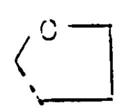 ; 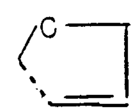

Ar für einen Arylrest mit 5 oder 6 carbocyclischen oder heterocyclischen Kettengliedern steht, ausgewählt unter den Thienyl-, Furyl-, Thiazolyl-, Pyrrolyl-, Oxazolyl-, Imidazolyl-, Pyrazolyl-, Triazolyl-(1,2,3 oder 1,2,4)-tetrazolyl-, Isothiazolyl-, Isoxazolyl-, Thiadiazolyl-, Phenyl-, Pyridinyl-, Pyridazinyl-, Pyrimidinyl-, Pyrazinyl-, Piperidinylresten,

wobei diese Reste gegebenenfalls durch einen oder mehrere gleiche oder verschiedene Substituenten substituiert sind, ausgewählt unter den Resten

- Halogen,
- Alkyl,
- Alkoxy,
- Alkylthio,
- Amino, Alkylamino, Dialkylamino, wobei ein jeder der Dialkylaminoreste gegebenenfalls in oxidierter Form vorliegt,
- Aminoalkyl,
- Dialkylaminoalkyl,
- Dialkylaminoalkoxy,
- Hydroxyl, Acetoxy, einem Rest der Formel

$$-O-\underset{\underset{O}{\parallel}}{C}-(CH_2)_nCO_2H,$$

  worin n = 2 bis 5,
- Acyl,

- freies oder verestertes Carboxy,
- Cyano,
- Trifluormethyl,
- Aryl oder Aralkyl,

sowie die Additionssalze der Produkte der Formel (I) mit Säuren und Basen.

**2.** Produkte der Formel (I), wie in Anspruch 1 definiert, worin der Ring A die Gruppe

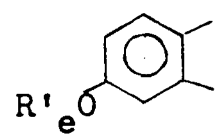

bedeutet, worin $R'_e$ für ein Wasserstoffatom oder einen Alkylrest mit 1 bis 4 Kohlenstoffatomen steht.

**3.** Produkte der Formel (I), wie in einem der Ansprüche 1 oder 2 definiert, worin Ar bedeutet:
(a) entweder einen Phenylrest, der gegebenenfalls durch einen oder mehrere Substituenten substituiert ist, ausgewählt unter den Halogen-, Alkyl-, Alkoxy- oder Alkylthioresten mit 1 bis 4 Kohlenstoffatomen, Amino-, Alkylamino-, Dialkyamino-, Dialkylaminoalkoxy-, Hydroxyl-, Acyl-, freien, veresterten oder in ein Salz überführten Carboxy-, Cyano-, Trifluormethyl-, Phenyl- oder Benzylresten, die ihrerseits gegebenenfalls durch einen oder mehrere Alkylreste mit 1 bis 4 Kohlenstoffatomen substituiert sind,
(b) oder einen heterocyclischen Rest, ausgewählt unter den Thienyl-, Furyl-, Thiazolyl-, Isothiazolyl-, Oxazolyl-, Isoxazolyl-, Thiadiazolyl-, Pyridinyl-, Piperidinylresten.

**4.** Produkte der Formel (I), wie in einem der Ansprüche 1 bis 3 definiert, worin $R_1$ einen gegebenenfalls geschützten oder acylierten Hydroxylrest bedeutet, $R_2$ für einen Alkyl-, Alkenyl- oder Alkinylrest steht, wobei ein jeder dieser Reste gegebenenfalls durch einen Rest substituiert ist, ausgewählt unter den Halogenen, den Hydroxy-, gegebenenfalls veresterten oder in ein Salz überführten Carboxy- oder Cyanoresten.

**5.** Produkte der allgemeinen Formel (I), wie in einem der Ansprüche 1 bis 4 definiert, mit den folgenden Bezeichnungen:
7α-[4-[2-(Dimethylamino)-ethoxy]-phenyl]-östra-1,3,5(10)-trien-3,17β-diol,
7α-[4-[2-(Dimethylamino)-ethoxy]-phenyl]-17β-hydroxy-östr-4-en-3-on,
7β-[4-[2-(Dimethylamino)-ethoxy]-phenyl]-östra-1,3,5(10)-trien-3,17β-diol,
7β-[4-(Dimethylamino)-ethoxy]-phenyl]-17β-hydroxy-östr-4-en-3-on,
7α-[4-[2-(Dimethylamino)-phenyl]-östra-1,3,5(10)-trien-3,17β-diol,
7α-(4-Methoxyphenyl)-östra-1,3,5(10)-trien-3,17β-diol,
17β-Hydroxy-7α-[4-(methylthio)-phenyl]-östr-4-en-3-on,
7α-[4-(Methylthio)-phenyl]-östra-1,3,5(10)-trien-3,17β-diol.

**6.** Verfahren zur Herstellung der Produkte der allgemeinen Formel (I), wie in Anspruch 1 definiert, dadurch gekennzeichnet, daß man ein Produkt der Formel (II)

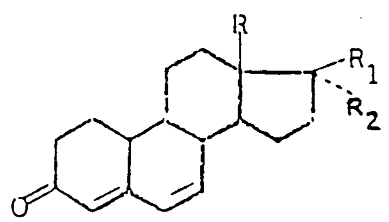

(II)

worin R, $R_1$ und $R_2$ die in Anspruch 1 angegebene Bedeutung besitzen, zunächst gegebenenfalls in Anwesenheit einer Lewissäure der Einwirkung

- entweder eines Produkts der Formel ($III_a$)

  Ar-Mg-Hal ($III_a$)

  worin Ar die in Anspruch 1 angegebene Bedeutung besitzt und Hal für ein Halogenatom steht, in Anwesenheit eines Kuprosalzes
- oder eines Produkts der Formel ($III_b$)

  $Ar_2CuLi$ ($III_b$)

- oder eines Produkts der Formel ($III_c$)

  $Ar_2CuCNLi_2$ ($III_c$)

unterzieht, um zu einem Produkt der Formel ($I_A$)

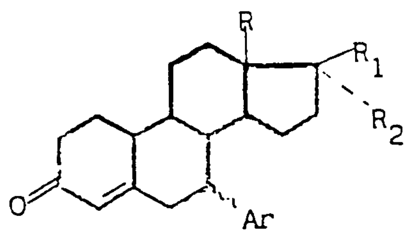

($I_A$)

gegebenenfalls in Form eines Gemisches der 7α- und 7β-Isomeren zu gelangen, welches man gewünschtenfalls trennt und das einem Produkt der Formel (I) entsprechende Produkt der Formel $I_A$, worin der Ring A für die Gruppe

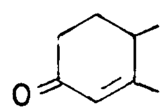

steht, man gewünschtenfalls mit einem Aromatisierungsmittel behandelt, um zu einem Produkt der Formel

($I_{B_1}$)

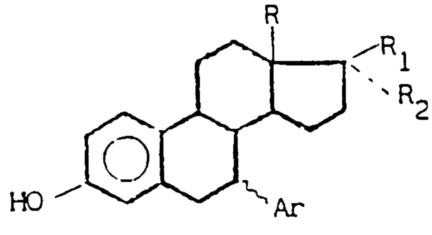

($I_{B_1}$)

worin Ar, R, $R_1$ und $R_2$ die in Anspruch 1 angegebene Bedeutung besitzen und das einem Produkt der Formel I entspricht, worin der Ring A die Gruppe

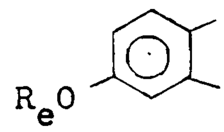

worin Re für ein Wasserstoffatom steht, bedeutet, zu gelangen, und das Produkt der Formel

$(I_{B_1})$

gewünschtenfalls mit einem Alkylierungsreagens behandelt, um zu einem Produkt der Formel

$(I_{B_2})$

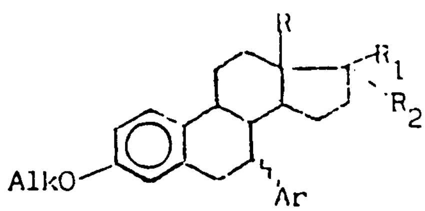

$(I_{B_2})$

worin Alk für einen gegebenenfalls substituierten Alkylrest mit 1 bis 6 Kohlenstoffatomen steht und das einem Produkt der Formel (I) entspricht, worin der Ring A die Gruppe

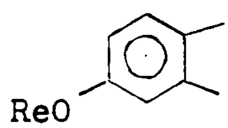

,

in der Re einen gegebenenfalls substituierten Alkylrest bedeutet, wiedergibt, zu gelangen, und man die Produkte der Formeln $(I_A)$,

$(I_{B_1})$ oder $(I_{B_2})$

entsprechend der Bedeutung der Substituenten $R_2$ und Ar gegebenenfalls der Einwirkung einer Base oder einer Säure unterzieht, um die entsprechenden Salze zu erhalten.

**7.** Verfahren gemäß Anspruch 6, dadurch gekennzeichnet, daß man ein Produkt der Formel $(I_C)$

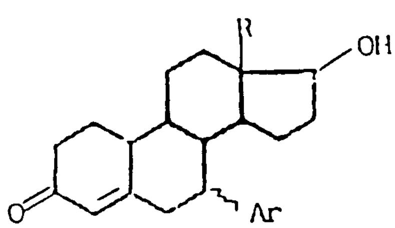

$(I_C)$

worin R und Ar die in Anspruch 1 angegebene Bedeutung besitzen und das einem Produkt der Formel ($I_A$) entspricht, worin $R_1$ einen Hydroxylrest wiedergibt und $R_2$ für ein Wasserstoffatom steht, der Einwirkung eines Reagens zur Blockierung der 3-Ketofunktion, z.B. eines Enolethers oder eines Ketals, unterzieht, um ein Produkt der Formel (IV)

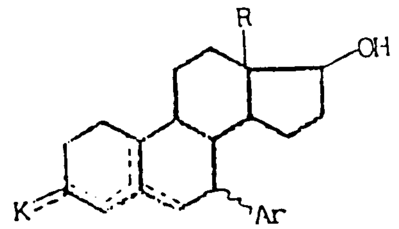

(IV)

worin K und die gestrichelten Linien eine geschützte Ketofunktion wiedergeben, vorzugsweise ein Produkt der Formeln ($IV_A$) oder ($IV_B$)

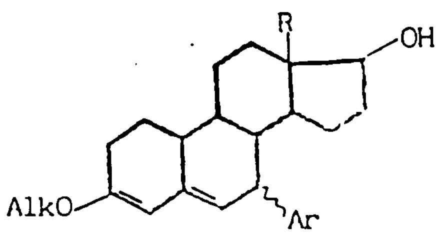

($IV_A$)

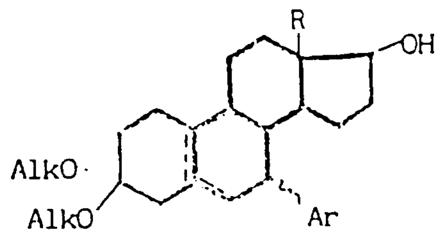

($VI_B$)

zu erhalten, worin Alk vorzugsweise einen Alkylrest wiedergibt und die gestrichelten Linien eine Doppelbindung in 5(6)- oder 5(10)-Stellung bedeuten, das Produkt der Formel (IV) mit einem Oxidationsreagens behandelt, um zu einem Produkt der Formel (V)

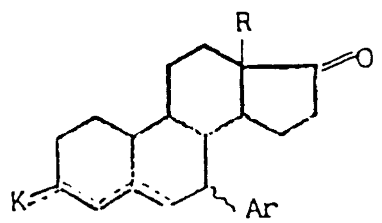

(V)

zu gelangen, mit dem man entweder ein organometallisches Derivat, das sich von einem Rest $R_2$ ableitet, der für einen Alkyl-, Alkenyl-, Alkinyl-Rest mit höchstens 8 Kohlenstoffatomen oder einen Aryl- oder Aralkylrest mit höchstens 15 Kohlenstoffatomen steht, wobei ein jeder dieser Alkyl-, Alkenyl-, Alkinyl-, Aryl- oder Aralkylreste gegebenenfalls substituiert ist, oder zunächst ein organometallisches Derivat, das sich von einem Reagens der Formel $-C{\equiv}C-CH_2OH$ ableitet, worin die Hydroxylfunktion gegebenenfalls geschützt ist, und danach in beliebiger Reihenfolge gewünschtenfalls mit einem Reagens zur Schutzgruppen-Abspaltung und mit einem Reagens für die vollständige oder teilweise Reduktion und hiernach mit einem Cyclisierungs- oder Oxidationsreagens oder zunächst mit einem Reagens der Formel

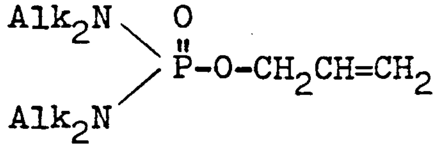

in Gegenwart einer starken Base umsetzt, um zu den Produkten der Formel ($VI_A$)

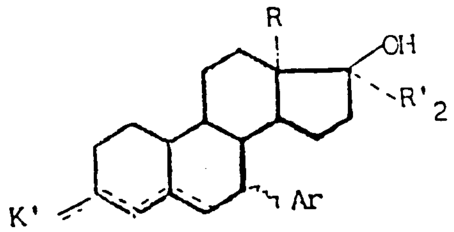

$(VI_A)$

worin $R'_2$ die Bedeutungen von $R_2$ mit Ausnahme der Bedeutung Wasserstoff besitzt und K' und die gestrichelten Linien eine 3-Keto-Δ4-funktion, die gegebenenfalls geschützt ist, wiedergeben, bzw. den Produkten der Formel $(VI_B)$

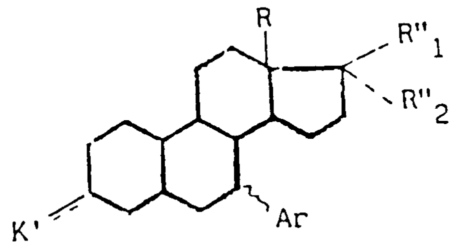

$(VI_B)$

zu gelangen, worin $R''_1$ und $R''_2$ gemeinsam einen Rest

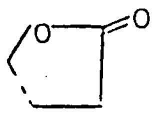

;

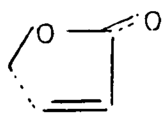

,

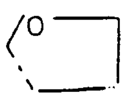

oder

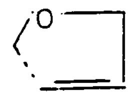

wiedergeben, die Produkte der Formeln $(VI_A)$ und $(VI_B)$ gegebenenfalls einem Reagens zur Schutzgruppen-Abspaltung unterzieht, wenn K' eine Schutzgruppe für die 3-Keto-Δ4-funktion bedeutet, um zu den Produkten der Formel $(I_D)$

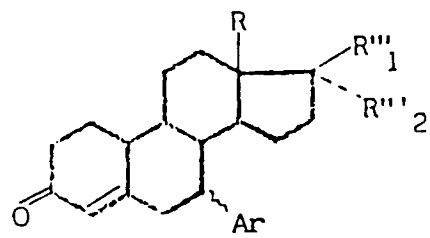

$(I_D)$

zu gelangen, worin $R'''_1$ und $R'''_2$ die vorstehend für $R_1$ und $R_2$ angegebenen Bedeutungen mit Ausnahme der Bedeutung $R_2$ = Wasserstoff besitzen, man die Produkte der Formel $(I_D)$ gegebenenfalls, wenn $R'''_1$ für einen Hydroxylrest steht, der Einwirkung eines Reagens zur Einführung einer Schutzgruppe, eines Acylierungsreagens oder eines Alkylierungsreagens unterzieht, um ein entsprechendes Produkt $(I'_D)$ zu erhalten, worin die 17β-OH-Funktion geschützt, acyliert oder alkyliert ist, und man das den Produkten der Formel $(I_A)$ entsprechende Produkt der Formeln $(I_D)$ und $(I'_D)$ gegebenenfalls, wie in Anspruch 1 angegeben, behandeln kann.

**8.** Als Arzneimittel die Produkte der allgemeinen Formel (I), wie in Anspruch 1 definiert, oder deren pharmazeutisch verträgliche Salze.

**9.** Als Arzneimittel die Produkte der allgemeinen Formel (I), wie in einem der Ansprüche 2 bis 5 definiert, oder deren pharmazeutisch verträgliche Salze.

**10.** Pharmazeutische Zusammensetzungen, enthaltend als Wirkstoff zumindest ein Arzneimittel gemäß einem der Ansprüche 8 oder 9.

**Patentansprüche für folgende Vertragsstaaten : ES, GR**

**1.** Verfahren zur Herstellung von Produkten der allgemeinen Formel (I)

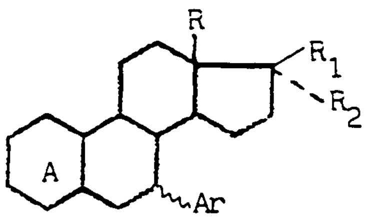

(I)

worin der Ring A eine der folgenden Strukturen besitzt:

(a) entweder bedeutet A die Gruppe

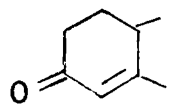

(b) oder A bedeutet die Gruppe

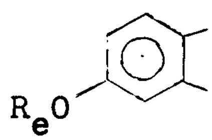

worin Re ein Wasserstoffatom, eine Alkylgruppe mit 1 bis 6 Kohlenstoffatomen, die gegebenenfalls substituiert ist, oder einen Acylrest bedeutet,

R für einen Methyl- oder Ethylrest steht,

$R_1$ eine Hydroxylgruppe bedeutet, die gegebenenfalls geschützt oder mit einem Acylrest, ausgewählt unter den Acetyl-, Chloracetyl- oder Trifluoracetylresten, acyliert ist, oder $R_1$ einen Alkoxyrest bedeutet,

$R_2$ ein Wasserstoffatom, einen Alkyl-, Alkenyl- oder Alkinylrest mit höchstens 8 Kohlenstoffatomen oder einen Aryl- oder Aralkylrest mit höchstens 15 Kohlenstoffatomen wiedergibt, wobei ein jeder dieser Alkyl-, Alkenyl-, Alkinyl-, Aryl- oder Aralkylreste gegebenenfalls durch einen oder mehrere gleiche oder verschiedene Substituenten substituiert ist, ausgewählt unter den Resten:

- Halogen,
- Alkoxy,
- Alkylthio,
- Amino, Alkylamino, Dialkylamino, wobei ein jeder der Dialkylaminoreste gegebenenfalls in oxidierter Form vorliegt,
- Aminoalkyl,
- Dialkylaminoalkyl,
- Dialkylaminoalkoxy,
- Hydroxyl, Acetoxy, einem Rest der Formel

$$-O-\underset{\underset{O}{\|}}{C}-(CH_2)_nCO_2H,$$

worin n = 2 bis 5,
- Acyl, - freies oder verestertes Carboxy,
- Cyano,
- Trifluormethyl,
- Aryl oder Aralkyl,

wobei die Aryl- und Aralkylreste, die $R_2$ wiedergeben kann, außerdem durch einen Alkylrest substituiert sein können, oder $R_1$ und $R_2$ gemeinsam einen Rest bilden, ausgewählt unter den Resten

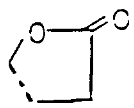 ; 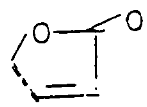 ; 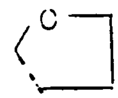 ; 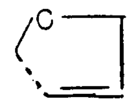

Ar für einen Arylrest mit 5 oder 6 carbocyclischen oder heterocyclischen Kettengliedern steht, ausgewählt unter den Thienyl-, Furyl-, Thiazolyl-, Pyrrolyl-, Oxazolyl-, Imidazolyl-, Pyrazolyl-, Triazolyl-(1,2,3 oder 1,2,4)-, Tetrazolyl-, Isothiazolyl-, Isoxazolyl-, Thiadiazolyl-, Phenyl-, Pyridinyl-, Pyridazinyl-, Pyrimidinyl-, Pyrazinyl-, Piperidinylresten,
wobei diese Reste gegebenenfalls durch einen oder mehrere gleiche oder verschiedene Substituenten substituiert sind, ausgewählt unter den Resten
- Halogen,
- Alkyl,
- Alkoxy,
- Alkylthio,
- Amino, Alkylamino, Dialkylamino, wobei ein jeder der Dialkylaminoreste gegebenenfalls in oxidierter Form vorliegt,
- Aminoalkyl,
- Dialkylaminoalkyl,
- Dialkylaminoalkoxy,
- Hydroxyl, Acetoxy, einem Rest der Formel

$$-O-\underset{\underset{O}{\|}}{C}-(CH_2)_nCO_2H,$$

worin n = 2 bis 5,
- Acyl,
- freies oder verestertes Carboxy,
- Cyano,
- Trifluormethyl,
- Aryl oder Aralkyl,

sowie der Additionssalze der Produkte der Formel (I) mit Säuren und Basen,
dadurch gekennzeichnet, daß man ein Produkt der Formel (II)

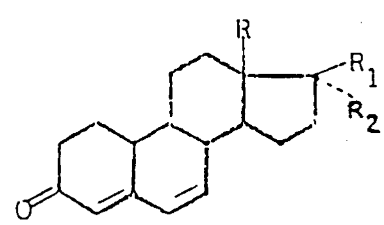

(II)

worin R, $R_1$ und $R_2$ die vorstehend angegebene Bedeutung besitzen, zunächst gegebenenfalls in Anwesenheit einer Lewissäure der Einwirkung
- entweder eines Produkts der Formel ($III_a$)

Ar-Mg-Hal (III$_a$)

worin Ar die vorstehend angegebene Bedeutung besitzt und Hal für ein Halogenatom steht, in Anwesenheit eines Kuprosalzes

- oder eines Produkts der Formel (III$_b$)

$Ar_2CuLi$ (III$_b$)

- oder eines Produkts der Formel (III$_c$)

$Ar_2CuCNLi_2$ (III$_c$)

unterzieht, um zu einem Produkt der Formel (I$_A$)

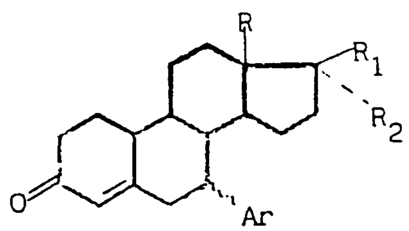

(I$_A$)

gegebenenfalls in Form eines Gemisches der 7α- und 7β-Isomeren zu gelangen, welches man gewünschtenfalls trennt und das einem Produkt der Formel (I) entsprechende Produkt der Formel I$_A$, worin der Ring A für die Gruppe

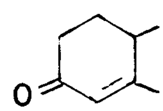

steht, man gewünschtenfalls mit einem Aromatisierungsmittel behandelt, um zu einem Produkt der Formel

(I$_{B_1}$)

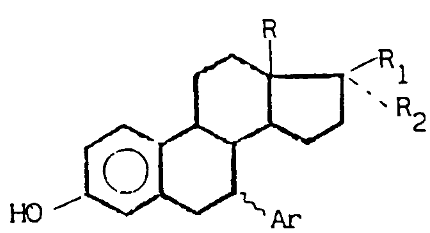

(I$_{B_1}$)

worin Ar, R, R$_1$ und R$_2$ die vorstehend angegebene Bedeutung besitzen und das einem Produkt der Formel I entspricht, worin der Ring A die Gruppe

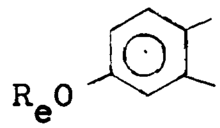

worin Re für ein Wasserstoffatom steht, bedeutet, zu gelangen, und das Produkt der Formel

$(I_{B_1})$

gewünschtenfalls mit einem Alkylierungsreagens behandelt, um zu einem Produkt der Formel

$(I_{B_2})$

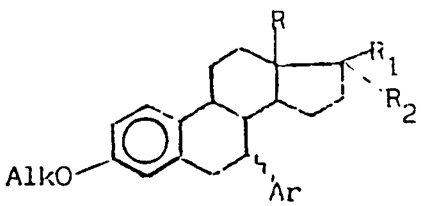

$(I_{B_2})$

worin Alk für einen gegebenenfalls substituierten Alkylrest mit 1 bis 6 Kohlenstoffatomen steht und das einem Produkt der Formel (I) entspricht, worin der Ring A die Gruppe

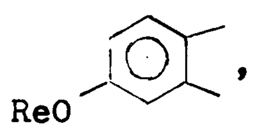

in der Re einen gegebenenfalls substituierten Alkylrest bedeutet, wiedergibt, zu gelangen, und man die Produkte der Formeln ($I_A$),

$(I_{B_1})$ oder $(I_{B_2})$

entsprechend der Bedeutung der Substituenten $R_2$ und Ar gegebenenfalls der Einwirkung einer Base oder einer Säure unterzieht, um die entsprechenden Salze zu erhalten.

**2.** Verfahren gemäß Anspruch 1, dadurch gekennzeichnet, daß man ein Produkt der Formel ($I_C$)

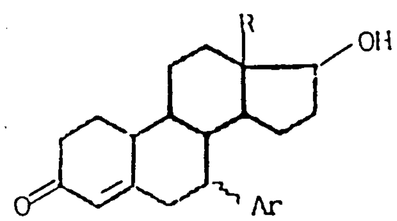

$(I_C)$

worin R und Ar die vorstehend angegebene Bedeutung besitzen und das einem Produkt der Formel ($I_A$)

entspricht, worin $R_1$ einen Hydroxylrest wiedergibt und $R_2$ für ein Wasserstoffatom steht, der Einwirkung eines Reagens zur Blockierung der 3-Ketofunktion, z.B. eines Enolethers oder eines Ketals, unterzieht, um ein Produkt der Formel (IV)

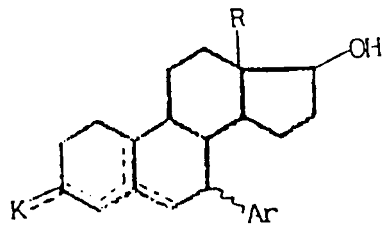

(IV)

worin K und die gestrichelten Linien eine geschützte Ketofunktion wiedergeben, vorzugsweise ein Produkt der Formeln ($IV_A$) oder ($IV_B$)

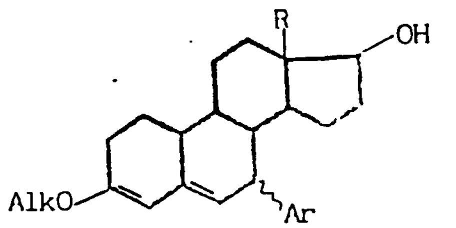

($IV_A$)

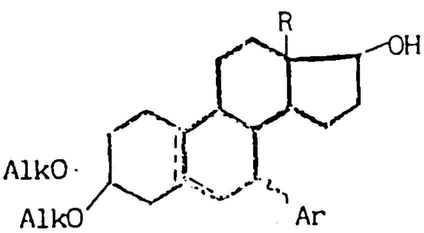

($VI_B$)

zu erhalten, worin Alk vorzugsweise einen Alkylrest wiedergibt und die gestrichelten Linien eine Doppelbindung in 5(6)- oder 5(10)-Stellung bedeuten, das Produkt der Formel (IV) mit einem Oxidationsreagens behandelt, um zu einem Produkt der Formel (V)

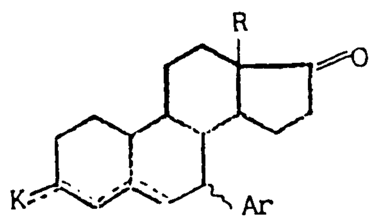

(V)

zu gelangen, mit dem man entweder ein organometallisches Derivat, das sich von einem Rest $R_2$ ableitet, der für einen Alkyl-, Alkenyl-, Alkinyl-Rest mit höchstens 8 Kohlenstoffatomen oder einen Aryl- oder Aralkylrest mit höchstens 15 Kohlenstoffatomen steht, wobei ein jeder dieser Alkyl-, Alkenyl-, Alkinyl-, Aryl- oder Aralkylreste gegebenenfalls substituiert ist, oder zunächst ein organometallisches Derivat, das sich von einem Reagens der Formel $-C{\equiv}C-CH_2OH$ ableitet, worin die Hydroxylfunktion gegebenenfalls geschützt ist, und danach in beliebiger Reihenfolge gewünschtenfalls mit einem Reagens zur Schutzgruppen-Abspaltung und mit einem Reagens für die vollständige oder teilweise Reduktion und hiernach mit einem Cyclisierungs- oder Oxidationsreagens oder zunächst mit einem Reagens der Formel

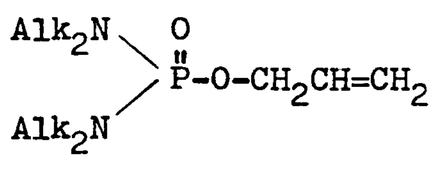

in Gegenwart einer starken Base umsetzt, um zu den Produkten der Formel ($VI_A$)

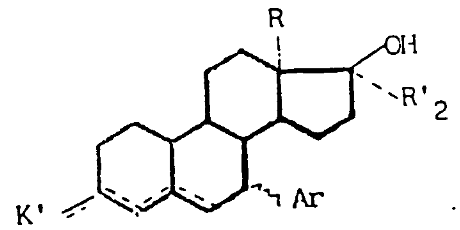

$(VI_A)$

worin $R'_2$ die Bedeutungen von $R_2$ mit Ausnahme der Bedeutung Wasserstoff besitzt und K' und die gestrichelten Linien eine 3-Keto-Δ4-funktion, die gegebenenfalls geschützt ist, wiedergeben, bzw. den Produkten der Formel $(VI_B)$

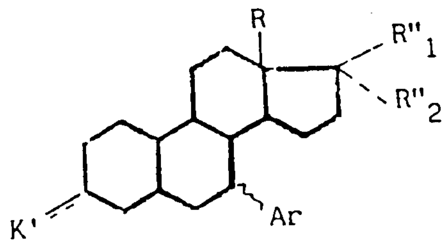

$(VI_B)$

zu gelangen, worin $R''_1$ und $R''_2$ gemeinsam einen Rest

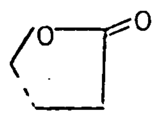 ; 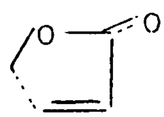 , 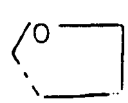 oder 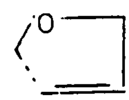

wiedergeben, die Produkte der Formeln $(VI_A)$ und $(VI_B)$ gegebenenfalls einem Reagens zur Schutzgruppen-Abspaltung unterzieht, wenn K' eine Schutzgruppe für die 3-Keto-Δ4-funktion bedeutet, um zu den Produkten der Formel $(I_D)$

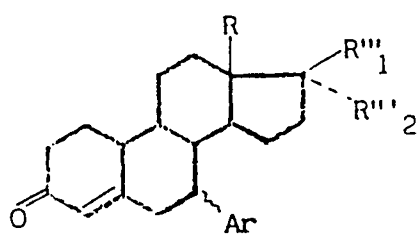

$(I_D)$

zu gelangen, worin $R'''_1$ und $R'''_2$ die vorstehend für $R_1$ und $R_2$ angegebenen Bedeutungen mit Ausnahme der Bedeutung $R_2$ = Wasserstoff besitzen, man die Produkte der Formel $(I_D)$ gegebenenfalls, wenn $R'''_1$ für einen Hydroxylrest steht, der Einwirkung eines Reagens zur Einführung einer Schutzgruppe, eines Acylierungsreagens oder eines Alkylierungsreagens unterzieht, um ein entsprechendes Produkt $(I'_D)$ zu erhalten, worin die 17β-OH-Funktion geschützt, acyliert oder alkyliert ist, und man das den Produkten der Formel $(I_A)$ entsprechende Produkt der Formeln $(I_D)$ und $(I'_D)$ gegebenenfalls, wie in Anspruch 1 angegeben, behandeln kann.

**3.** Verfahren gemäß Anspruch 1 oder 2, dadurch gekennzeichnet, daß man Produkte der Formel (I) herstellt, worin der Ring A die Gruppe

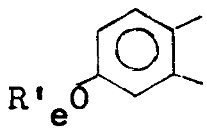

bedeutet, worin $R'_e$ für ein Wasserstoffatom oder einen Alkylrest mit 1 bis 4 Kohlenstoffatomen steht.

**4.** Verfahren gemäß einem der Ansprüche 1 bis 3, dadurch gekennzeichnet, daß man von einem Produkt der Formel ($III_a$), ($III_b$) oder ($III_c$) oder einem Produkt der Formel ($I_C$) ausgeht, worin Ar bedeutet:
(a) entweder einen Phenylrest, der gegebenenfalls durch einen oder mehrere Substituenten substituiert ist, ausgewählt unter den Halogen-, Alkyl-, Alkoxy- oder Alkylthioresten mit 1 bis 4 Kohlenstoffatomen, Amino-, Alkylamino-, Dialkylamino-, Dialkylaminoalkoxy-, Hydroxyl-, Acyl-, freien, veresterten oder in ein Salz überführten Carboxy-, Cyano-, Trifluormethyl-, Phenyl- oder Benzylresten, die ihrerseits gegebenenfalls durch einen oder mehrere Alkylreste mit 1 bis 4 Kohlenstoffatomen substituiert sind,
(b) oder einen heterocyclischen Rest, ausgewählt unter den Thienyl-, Furyl-, Thiazolyl-, Isothiazolyl-, Oxazolyl-, Isoxazolyl-, Thiadiazolyl-, Pyridinyl-, Piperidinylresten.

**5.** Verfahren gemäß Anspruch 1 oder 3, dadurch gekennzeichnet, daß man von einem Produkt der Formel (II) ausgeht, worin $R_1$ einen gegebenenfalls geschützten oder acylierten Hydroxylrest bedeutet, $R_2$ für einen Alkyl-, Alkenyl- oder Alkinylrest steht, wobei ein jeder dieser Reste gegebenenfalls durch einen Rest substituiert ist, ausgewählt unter den Halogenen, den Hydroxy-, gegebenenfalls veresterten oder in ein Salz überführten Carboxy- oder Cyanoresten.

**6.** Verfahren gemäß einem der Ansprüche 1 bis 5, dadurch gekennzeichnet, daß man herstellt:
7α-[4-[2-(Dimethylamino)-ethoxy)-phenyl]-östra-1,3,5(10)-trien-3,17β-diol,
7α-[4-[2-(Dimethylamino)-ethoxy]-phenyl]-17β-hydroxy-östr-4-en-3-on,
7β-[4-[2-(Dimethylamino)-ethoxy]-phenyl]-östra-1,3,5(10)-trien-3,17β-diol,
7β-[4-(Dimethylamino)-ethoxy]-phenyl]-17β-hydroxy-östr-4-en-3-on,
7α-[4-[2-(Dimethylamino)-phenyl]-östra-1,3,5(10)-trien-3,17β-diol,
7α-(4-Methoxyphenyl)-östra-1,3,5(10)-trien-3,17β-diol,
17β-Hydroxy-7α-[4-(methylthio)-phenyl]-östr-4-en-3-on,
7α-[4-(Methylthio)-phenyl]-östra-1,3,5(10)-trien-3,17β-diol.

**7.** Verfahren zur Herstellung von pharmazeutischen Zusammensetzungen, dadurch gekennzeichnet, daß man als Wirkstoff zumindest eine der Verbindungen der Formel (I), wie in Anspruch 1 definiert, oder zumindest eines ihrer pharmazeutisch verträglichen Additionssalze in eine für diese Verwendung bestimmte Form überführt.

**8.** Verfahren zur Herstellung von pharmazeutischen Zusammensetzungen, dadurch gekennzeichnet, daß man als Wirkstoff zumindest eine der Verbindungen der Formel (I), wie in Anspruch 11 definiert, oder zumindest eines ihrer pharmazeutisch vertraglichen Salze in eine für diese Verwendung bestimmte Form überführt.